(19) Europäisches Patentamt
European Patent Office
Office européen des brevets

(11) **EP 3 293 270 B1**

(12) **EUROPEAN PATENT SPECIFICATION**

(45) Date of publication and mention
of the grant of the patent:
**25.09.2019 Bulletin 2019/39**

(51) Int Cl.:
*C12Q 1/6869* (2018.01)    *C12Q 1/6827* (2018.01)
*C12Q 1/6883* (2018.01)

(21) Application number: **15891099.2**

(22) Date of filing: **06.05.2015**

(86) International application number:
**PCT/CN2015/078422**

(87) International publication number:
**WO 2016/176847 (10.11.2016 Gazette 2016/45)**

(54) **REAGENT KIT, APPARATUS, AND METHOD FOR DETECTING CHROMOSOME ANEUPLOIDY**

REAGENZIENKIT, VORRICHTUNG UND VERFAHREN ZUR ERKENNUNG VON CHROMOSOMENANEUPLOIDIE

KIT DE RÉACTIFS, APPAREIL ET PROCÉDÉ POUR LA DÉTECTION DE L'ANEUPLOÏDIE CHROMOSOMIQUE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(43) Date of publication of application:
**14.03.2018 Bulletin 2018/11**

(73) Proprietors:
• **Zhejiang Annoroad Bio-Technology Co., Ltd. Choujiang Subdistrict Yiwu City, Zhejiang Province, 322000 (CN)**
• **Annoroad Gene Technology (Beijing) Co., Ltd Beijing 100176 (CN)**

(72) Inventors:
• **CHEN, Chongjian Beijing 100176 (CN)**
• **LIANG, Junbin Beijing 100176 (CN)**
• **XUAN, Zhaoling Beijing 100176 (CN)**
• **LI, Dawei Beijing 100176 (CN)**

(74) Representative: **Huenges, Martin Maiwald Patentanwalts- und Rechtsanwaltsgesellschaft mbH Elisenhof Elisenstraße 3 80335 München (DE)**

(56) References cited:
WO-A1-2014/040206     WO-A1-2016/116033
CN-A- 102 985 561      CN-A- 104 789 466
CN-A- 104 789 686      US-A1- 2014 100 792

• **BAYINDIR B ET AL: "Noninvasive prenatal testing using a novel analysis pipeline to screen for all autosomal fetal aneuploidies improves pregnancy management", EUROPEAN JOURNAL OF HUMAN GENETICS, vol. 23, no. 10, 14 January 2015 (2015-01-14), pages 1286-1293, XP055378014, ISSN: 1018-4813, DOI: 10.1038/ejhg.2014.282**
• **CHIU R W K ET AL: "Noninvasive prenatal diagnosis of fetal chromosomal aneuploidy by massively parallel genomic sequencing of DNA in maternal plasma", PROCEEDINGS NATIONAL ACADEMY OF SCIENCES PNAS, vol. 105, no. 51, 23 December 2008 (2008-12-23), pages 20458-20463, XP055284693, US ISSN: 0027-8424, DOI: 10.1073/pnas.0810641105 -& R. W. K. CHIU ET AL: "Supplementary Information for XP002620454 (Title: Noninvasive prenatal diagnosis of fetal chromosomal aneuploidy by massively parallel genomic sequencing of DNA in maternal plasma)", PROCEEDINGS NATIONAL ACADEMY OF SCIENCES PNAS, vol. 105, no. 51, 10 December 2008 (2008-12-10), pages 20458-20463, XP055024153, US ISSN: 0027-8424, DOI: 10.1073/pnas.0810641105**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- FAN H C ET AL: "Non-invasive prenatal measurement of the fetal genome", NATURE, vol. 487, no. 7407, 4 July 2012 (2012-07-04), pages 320-324, XP055109901, ISSN: 0028-0836, DOI: 10.1038/nature11251
- SNYDER M S ET AL: "Copy-Number Variation and False Positive Prenatal Aneuploidy Screening Results", NEW ENGLAND JOURNAL OF MEDICINE, THE - NEJM -, vol. 372, no. 17, 23 April 2015 (2015-04-23), pages 1639-1645, XP055328136, US ISSN: 0028-4793, DOI: 10.1056/NEJMoa1408408
- CHANDRANANDA D ET AL: "Investigating and Correcting Plasma DNA Sequencing Coverage Bias to Enhance Aneuploidy Discovery", PLOS ONE, vol. 9, no. 1, 29 January 2014 (2014-01-29), page e86993, XP055118849, DOI: 10.1371/journal.pone.0086993
- CHIU, R.W.K. ET AL.: 'Noninvasive Prenatal Diagnosis of Fetal Chromosomal Aneuploidy by Massively Parallel Genomic Sequencing of DNA in Maternal Plasma' PNAS vol. 105, no. 51, 23 December 2008, pages 20458 - 20463, XP055284693
- MATTHEW, W. ET AL.: 'Copy-Number Variation and False Positive Prenatal Aneuploidy Screening Results' N ENGL J MED. vol. 372, no. 17, 23 April 2015, pages 1639 - 1645, XP055328136

**Description**

FIELD OF THE INVENTION

The present invention relates to the biomedical field, more particularly, to a kit, an apparatus and a method for detecting chromosome aneuploidy.

BACKGROUND OF THE INVENTION

It has been 20 years since cell-free fetal DNA (cff-DNA) was found by Lo in 1997, which has provided possibility for varieties of non-invasive prenatal testing (NIPT). NIPT is advantageous in two aspects: on the one hand, NIPT will not cause any miscarriage risk, but the invasive manners including amniocentesis and cordocentesis for chromosome karyotype analysis will bring about 1/200 miscarriage risk, and there were researches indicating that cordocentesis may also cause fetus position tilted; on the other hand, NIPT can be performed as early as the 8th week of pregnancy, which provides earlier risk evaluation so as to decrease needs of induced labour for pregnant women.

These advantages lead to NIPT relevant methods developing rapidly and being widely applied as well. In current, there have been NIPT for fetal chromosome aneuploidy detection, NIPT for fetal single gene diseases, NIPT for fragments with copy number variations (CNV) in fetus, NIPT for fetal whole genome, NIPT for fetal paternity test and the like.

At present, among all of the NIPT applications, the most widely used and developed one is the fetal chromosome aneuploidy detection. In a number of methods for fetal chromosome aneuploidy detection, Chui's invention based on massively parallel sequencing (MPS) in 2008 is considered to be the most suitable one for clinical use, which has already exhibited its robustness. For Down syndrome, the false positive rate (FPR) can reach up to 0.443%, and the false negative rate (FNR) is as low as 0.004%; for Edward's syndrome, the FPR is 0.22%, and the FNR is 0.025%.

Non-invasive prenatal testing using an analysis pipeline to screen for all fetal aneuploidies has been described by Bayindir et al. (2015), Eur J Hum Gen, vol. 23(10), p. 1286-129.

Although above methods had achieved such low error rates, risks based on wrong judgments still exist. Therefore, improvement for existing methods is in demand so as to decrease the error rate of detection as low as possible.

SUMMARY OF THE INVENTION

The main object of the present application is to provide a kit, an apparatus and a method for detecting chromosome aneuploidy so as to reduce the false positive rate of the detection.

In order to achieve above object, according to one aspect of the present application, provided is a method for detecting chromosome aneuploidy, which includes the following steps of:

high-throughput sequencing of the peripheral blood cell-free DNA from a pregnant woman to be tested to produce sequencing data comprising all of the chromosomes;

calculating coverage statistics for all of the chromosomes with the sequencing data by segmenting the chromosomes into windows so as to produce a pre-correction coverage of each chromosome;

performing a Z-test on the number of unique sequences in each window of the pregnant woman to produce a $Z_{CNV}$ value and then locating chromosomal fragments with copy number variations of the pregnant woman on the basis of the magnitude of the $Z_{CNV}$ value; wherein chromosomal fragments with copy number variations of the pregnant woman are 300 kb or more in length and have $Z_{CNV}$ values of the chromosome fragments greater than or equal to 4 or less than or equal to -4 in at least 80% of the total windows within the fragments which are 300 kb or more in length,

correcting the pre-correction coverage of each chromosome by utilizing the impact of the fragments with copy number variations of the pregnant woman on the pre-correction coverage of each chromosome to produce the corrected coverage of each chromosome; and

performing a Z-test for each chromosome by using the corrected coverage of each chromosome to obtain a $Z_{aneu}$ value, and determining chromosome aneuploidy based on whether the absolute value *of* $Z_{aneu}$ is greater than or equal to 3; wherein if the absolute value *of* $Z_{aneu}$ is greater than or equal to 3, chromosome aneuploidy is determined ;

wherein the impact of the fragments with copy number variations of the pregnant woman to be tested on the pre-correction coverage of each chromosome is represented by a parameter $\alpha$,

when the fetus inherits the fragments with copy number variations from the mother, the parameter $\alpha$ is calculated as formula (1):

$$\alpha = \frac{(m-n)\cdot 2 + n\cdot cn}{m\cdot 2} \quad\ldots\ldots\ldots\ldots\ldots\ldots\ldots\ldots\ldots\ldots \quad (1)$$

when the fetus does not inherit the fragments with copy number variations from the mother, the parameter $\alpha$ is calculated as formula (2):

$$\alpha = \frac{(m-n)\cdot 2 + f\cdot n\cdot 2 + (1-f)\cdot n\cdot cn}{m\cdot 2} \quad\ldots\ldots\ldots\ldots\ldots \quad (2)$$

wherein in formula (1) and formula (2), m represents the effective length of the chromosome in which the fragments with copy number variations occur, in the unit of Mb; and n represents the length of the fragments with copy number variations of the pregnant woman to be tested, in the unit of Mb; cn represents the copy number of the fragments with copy number variations found in the pregnant woman to be tested;

wherein in formula (2), f represents the fraction of the cell-free fetal DNA existing in the peripheral blood cell-free DNA of the pregnant woman to be tested, and the fraction f of the cell-free fetal DNA is assumed to be less than 50%;

correcting the pre-correction coverage of each chromosome by using $x' = \dfrac{\hat{x}}{\alpha}$,

wherein $\hat{x}$ represents the pre-correction coverage of each chromosome and x' represents the corrected coverage of each chromosome.

Furthermore, the coverage statistics is calculated by segmenting all of the chromosomes in the sequencing data into windows with equal sizes so as to produce the pre-correction coverage of each chromosome.

In addition, the length of each window is 100 kb, and the overlapping ratio between two adjacent windows is 50%.

Further, the step of performing a Z-test on the number of unique sequences in each window of the pregnant woman to be tested to produce the $Z_{CNV}$ value and then locating chromosomal fragments with copy number variations of the pregnant woman to be tested on the basis of the magnitude of the $Z_{CNV}$ value further includes the steps of:

counting the number of the unique sequences in each window according to the sequencing depth of each sequence in the sequencing data;

calculating the number of the unique sequences in each window according to the GC content and the mapping rate of teach chromosome to obtain the pre-correction coverage of the number of the unique sequences in each window; and

normalizing the pre-correction coverage of the number of the unique sequences in each window to obtain the $Z_{CNV}$ value of the number of the unique sequences in each window and determining whether the pregnant woman to be tested has chromosomal fragments with copy number variations on the basis of the magnitude of the $Z_{CNV}$ value; if there is a fragment which is 300 kb or more in length in the sequencing data, and within the fragment which is 300 kb or more in length, the $Z_{CNV}$ values of the numbers of the unique sequences in at least 80% of the total windows are greater than or equal to 4 or less than or equal to -4, then the fragments which are 300 kb or more in length are determined to be the fragments with copy number variations of the pregnant woman to be tested.

Moreover, for the step of performing a Z-test for each chromosome by using the corrected coverage of each chromosome to obtain the $Z_{aneu}$ value, the $Z_{aneu}$ value is calculated as:

$$Z_{aneu} = \frac{x' - \overline{x}}{s}$$

wherein $\overline{x}$ represents the pre-correction coverage obtained from a known negative sample population according to a LOESS algorithm; s represents the standard error of $(x'\text{-}\overline{x})$ in the negative sample population.

In order to achieve the above object, in another aspect of the present application, provided is an apparatus for detecting chromosome aneuploidy, which includes the following modules:

a sequencing data detection module: for high-throughput sequencing of the peripheral blood cell-free DNA of a pregnant woman to be tested to produce the sequencing data comprising all of the chromosomes;

a first coverage calculation module: for calculating coverage statistics of all of the chromosomes with the sequencing data by segmenting the chromosomes into windows so as to produce a pre-correction coverage of each chromosome;

a $Z_{CNV}$ value calculation module: for calculating the $Z_{CNV}$ value on the number of unique sequences in each window of the pregnant woman;

a fragments with copy number variations search module: for searching the fragment that is 300 kb or more in length in the sequencing data and which has the $Z_{CNV}$ values of the chromosome fragments greater than or equal to 4 or less than or equal to -4 in at least 80% of the total windows;

a fragments with copy number variations determination module: for determining fragments in the sequencing data that are 300 kb or more in length and which have $Z_{CNV}$ values of the chromosome fragments greater than or equal to 4 or less than or equal to -4 in at least 80% of the total windows as the fragments with copy number variations of the pregnant woman;

a first α calculation module: for calculating a parameter α according to formula (1) in the case where the fetus inherits the fragments with copy number variations from the mother, wherein the parameter α represents the impact of the fragments with copy number variations of the pregnant woman on the pre-correction coverage of each chromosome;

wherein in formula (1) m represents the effective length of the chromosome in which the fragments with copy number variations occur, in the unit of Mb; and n represents the length of the fragments with copy number variations of the pregnant woman, in the unit of Mb; cn represents the copy number of the fragments with copy number variations found in the pregnant woman;

$$\alpha = \frac{(m-n)\cdot 2 + n\cdot cn}{m\cdot 2} \qquad \dots\dots\dots\dots\dots\dots\dots\dots\dots\dots\dots \; (1)$$

a second α calculation module: for calculating a parameter α according to formula (2) in the case where the fetus does not inherit the fragments with copy number variations from the mother, wherein the parameter α is calculated according to formula (2),

$$\alpha = \frac{(m-n)\cdot 2 + f\cdot n\cdot 2 + (1-f)\cdot n\cdot cn}{m\cdot 2} \qquad \dots\dots\dots\dots\dots \; (2)$$

wherein in formula (2), m represents the effective length of the chromosome in which the fragments with copy number variations occur, in the unit of Mb; and n represents the length of the fragments with copy number variations of the pregnant woman, in the unit of Mb; cn represents the copy number of the fragments with copy number variations found in the pregnant woman; f represents the fraction of the cell-free fetal DNA existing in the peripheral blood cell-free DNA of the pregnant woman, and the fraction f of the cell-free fetal DNA is assumed to be less than 50%;

$$x' = \frac{\hat{x}}{\alpha}.$$

a correction module: for correcting the pre-correction coverage of each chromosome by using: to produce the corrected coverage of each chromosome; wherein $\hat{x}$ represents the pre-correction coverage of each chromosome and x' represents the corrected coverage of each chromosome;

a second coverage calculation module: for calculating the $Z_{aneu}$ value of each chromosome by using the corrected coverage of each chromosome;

$Z_{aneu}$ value determination module: for determining, whether the absolute $Z_{aneu}$ value is greater than or equal to 3;

a chromosome aneuploidy confirmation module: for confirming the chromosome has aneuploidy in the case where the absolute $Z_{aneu}$ value is greater than or equal to 3.

Further, the first coverage calculation module includes:

a chromosome window segmentation sub-module: for segmenting all of the chromosomes in the sequencing data into windows with equal size;
a first coverage calculation sub-module: for calculating coverage statistics in the form of windows with equal size to produce the pre-correction coverage of each chromosome.

In addition, the length of each window in the chromosome window segmentation sub-module is 100 kb, and the overlapping ratio between two adjacent windows is 50%.

Furthermore, the $Z_{CNV}$ value calculation module includes:

a unique sequence counting unit: for counting the number of the unique sequences in each window according to the sequencing depth of each sequence in the sequencing data;
a unique sequence coverage calculation unit: for calculating the number of the unique sequences in each window according to the GC content and the mapping rate of each chromosome to obtain the pre-correction coverage of the number of the unique sequences in each window; and
a unique sequence $Z_{CNV}$ value calculation unit: for normalizing the pre-correction coverage of the number of the unique sequences in each window to obtain the $Z_{CNV}$ value of the number of the unique sequences in each window.

Additionally, in the second coverage calculation module, the $Z_{aneu}$ value is calculated as:

$$Z_{aneu} = \frac{x^{'} - \overline{x}}{s}$$

wherein $\overline{x}$ is the pre-correction coverage obtained from a known negative sample population according to a LOESS algorithm; s represents the standard error of $(x'\text{-}\overline{x})$ in the negative sample population.

According to another aspect of the present application, a kit is provided for detecting chromosome aneuploidy, including:

the detection reagents and a detection device: for high-throughput sequencing of the peripheral blood cell-free DNA from a pregnant woman to be tested to produce the sequencing data containing all of the chromosomes;
a first coverage calculation device: for calculating coverage statistics of all of the chromosomes with the sequencing data by segmenting the chromosomes into windows so as to produce a pre-correction coverage of each chromosome;
a $Z_{CNV}$ value calculation device: for performing a Z-test on the number of unique sequences in each window from the pregnant woman to be tested to obtain the $Z_{CNV}$ value;
a fragments with copy number variations search device: for searching the fragments in the sequencing data that are 300 kb or more in length and which have $Z_{CNV}$ values of the chromosome fragments greater than or equal to 4 or less than or equal to -4 in at least 80% of the total windows;
a fragments with copy number variations determination device: for obtaining the fragments with copy number variations of the pregnant woman to be tested on the basis of the magnitude of the $Z_{CNV}$ value;
a first α calculation device: for calculating a parameter α according to formula (1) in the case where the fetus inherits the fragments with copy number variations from the mother, wherein the parameter α represents the impact of the fragments with copy number variations of the pregnant woman to be tested on the pre-correction coverage of each chromosome;

$$\alpha = \frac{(m-n) \cdot 2 + n \cdot cn}{m \cdot 2} \quad \dots\dots\dots\dots\dots\dots\dots\dots\dots\dots (1)$$

wherein m represents the effective length of the chromosome in which the fragments with copy number variations occur, in the unit of Mb; and n represents the length of the fragments with copy number variations of the pregnant woman to be tested, in the unit of Mb; cn represents the copy number of the fragments with copy number variations found in the pregnant woman;
a second α calculation device: for calculating a parameter α according to formula (2) in the case where the fetus does not inherit the fragments with copy number variations from the mother, wherein the parameter α is calculated according to formula (2)

$$\alpha = \frac{(m-n)\cdot 2 + f\cdot n\cdot 2 + (1-f)\cdot n\cdot cn}{m\cdot 2} \qquad\ldots\ldots\ldots\ldots (2)$$

wherein m represents the effective length of the chromosome in which the fragments with copy number variations occur, in the unit of Mb; and n represents the length of the fragments with copy number variations of the pregnant woman, in the unit of Mb; cn represents the copy number of the fragments with copy number variations found in the pregnant woman; f represents the fraction of the cell-free fetal DNA existing in the peripheral blood cell-free DNA of the pregnant woman, and the fraction f of the cell-free fetal DNA is assumed to be less than 50%; a correction device: for correcting the pre-correction coverage of each chromosome by using:

$$x' = \frac{\hat{x}}{\alpha}.$$

to produce the corrected coverage of each chromosome; wherein $\hat{x}$ represents the pre-correction coverage of each chromosome and x' represents the corrected coverage of each chromosome; a second coverage calculation device: for calculating the $Z_{aneu}$ value of each chromosome by using the corrected coverage of each chromosome; $Z_{aneu}$ value determination device: for determining whether the absolute $Z_{aneu}$ value is greater than or equal to 3; a chromosome aneuploidy confirmation device: for confirming chromosome aneuploidy in the case where the absolute $Z_{aneu}$ value is greater than or equal to 3.

Further, the first coverage calculation device includes:

a chromosome window segmentation component: for segmenting all of the chromosomes in the sequencing data into windows with equal size; a first coverage calculation component: for calculating the coverage statistics in the form of windows with equal size to produce the pre-correction coverage of each chromosome.

Furthermore, the size of each window in the chromosome window segmentation component is 100 kb, and the overlapping ratio between two adjacent windows is 50%.

In addition, the $Z_{CNV}$ value calculation device includes:

a unique sequence counting component: for counting the number of the unique sequences in each window according to the sequencing depth of each sequence in the sequencing data; a unique sequence coverage calculation component: for calculating the number of the unique sequences in each window according to the GC content and the mapping rate of each chromosome to obtain the pre-correction coverage of the number of the unique sequences in each window; and a unique sequence $Z_{CNV}$ value calculation component: for normalizing the pre-correction coverage of the number of the unique sequences in each window to obtain the $Z_{CNV}$ value of the number of the unique sequences in each window.

Additionally, in the second coverage calculation device, the $Z_{aneu}$ value is calculated as:

$$Z_{aneu} = \frac{x' - \overline{x}}{s}$$

wherein $\overline{x}$ is the pre-correction coverage obtained from a known negative sample population according to a LOESS algorithm; s represents the standard error of ($x'-\overline{x}$) in the negative sample population.

According to the technical solution of the present invention, by screening the fragments with copy number variations occurring on the chromosome of the mother, and by determining chromosome aneuploidy based on removing the impact of the copy number variation on the coverage of each chromosome, thereby the corrected coverage of each chromosome can be obtained. By utilizing the corrected coverage to calculate and determine the chromosome aneuploidy, the present invention can achieve a more accurate result.

DESCRIPTION OF FIGURES

The accompanying figures are intended to provide a further understanding of the present application, and the illustrative examples of the present application and the description thereof are intended to explain the present application, which should not be construed as limiting the scope of the present application. In the figures:

Figure 1 shows a flow diagram of a method for detecting chromosome aneuploidy according to a typical embodiment of the present application;

Figure 2 shows a schematic diagram of an apparatus for detecting chromosome aneuploidy according to a typical embodiment of the present application;

Figures 3A, 3B and 3C show the corrected results indicating aneuploidy detection of chromosome 13, chromosome 18 and chromosome 21, respectively according to Example 1 of the present application;

Figure 4 shows the corrected result indicating aneuploidy of samples EK01875 and BD01462 on chromosome 21 according to Example 2 of the present application;

Figure 5 shows the corrected result indicating aneuploidy detection of sample EK01875 on chromosome 21 according to Example 3 of the present application; and

Figure 6 shows the corrected result indicating aneuploidy detection of sample BD01462 on chromosome 21 according to Example 4 of the present application.

DETAILED DESCRIPTION OF THE INVENTION

It is to be noted that the features in the embodiments and examples of the present invention can be combined with each other in a non-conflicting way. Hereinafter, the present invention will be described in detail with reference to the embodiments.

In this invention, $Z_{CNV}$ or $Z_{aneu}$ refers to the statistic value calculated by the Z-test, a method for testing mean difference of samples with large size (i.e. the sample size is greater than 30). It applies standard normal distribution theory to analyze the probability of occurrence of differences so as to conclude whether the difference between two averages is significant.

Mapping rate refers to a ratio obtained by aligning the sequencing sequence within the window to the reference sequence in the genome. Since the sequencing sequences in the windows may be aligned to multiple sites of the reference sequence in the genome but not an unique sequence, the mapping rate in the window is larger than that of an unique sequence.

It is to be noted that by extensive analysis, the applicant of the present invention has found that there are at least three possibilities causing misjudgments of NIPT through conventional methods:

First, Lo found that cff-DNA was derived from placenta in 1998, which means that if confined placental mosaicism (CPM) appears, it will be difficult to accurately estimate the situation of the fetus by NIPT and the results more likely to be inaccurate; second, if CNV exists in the pregnant woman herself, the method which is based on the MPS statistical coverage and is also converted to the Z value will be inaccurate. Therefore, when repeat fragments present in the pregnant woman, the relative numbers of unique sequences aligned to the chromosomes will increase, and the Z value will also increase as the increase of the coverage, thereby increasing the risk of false positives. Conversely, if there is a fragment deletion in the pregnant woman, the Z value will decrease, thereby increasing the risk of false negatives. Moreover, some of the previous studies have also shown that confined placental mosaicism (CPM) and copy number variation (CNV) are major reasons for false positive. Finally, during the calculation of the chromosome coverage and the correction of the coverage by GC content, there may be data fluctuation, thereby resulting in errors.

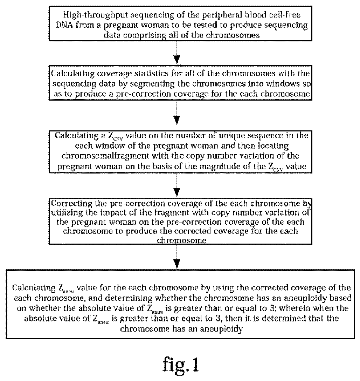

To this end, based on a comprehensive analysis for above-mentioned reasons for the errors, the present invention proposes a method for detecting chromosome aneuploidy, as shown in Fig. 1, which includes the steps of:

high-throughput sequencing of the peripheral blood cell-free DNA from a pregnant woman to be tested to produce sequencing data comprising all of the chromosomes;

calculating coverage statistics for all of the chromosomes with the sequencing data by segmenting the chromosomes into windows so as to produce a pre-correction coverage of each chromosome;

calculating a $Z_{CNV}$ value on the number of unique sequences in each window of the pregnant woman and then locating fragments with copy number variations of the pregnant woman on the basis of the magnitude of the $Z_{CNV}$ value; wherein the chromosomal fragments with copy number variations of the pregnant woman are 300 kb or more in length in the sequencing data and have $Z_{CNV}$ values of the chromosome fragments greater than or equal to 4 or less than or equal to -4 in at least 80% or more of the total windows among the fragment which is 300 kb or more in length,

correcting the pre-correction coverage of each chromosome by utilizing the impact of the fragments with copy number variations of the pregnant woman on the pre-correction coverage of each chromosome to produce the corrected coverage for the chromosome; and

using the corrected coverage of each chromosome to obtain the $Z_{aneu}$ value for each chromosome, and determining chromosome aneuploidy based on whether the absolute value of $Z_{aneu}$ is greater than or equal to 3; wherein if the absolute value of $Z_{aneu}$ is greater than or equal to 3, chromosome aneuploidy is determined;

wherein the impact of the fragments with copy number variations of the pregnant woman to be tested on the pre-correction coverage of each chromosome is represented by a parameter $\alpha$,

when the fetus inherits the fragments with copy number variations from the mother, the parameter $\alpha$ is calculated as formula (1):

$$\alpha = \frac{(m-n)\cdot 2 + n \cdot cn}{m \cdot 2} \quad\ldots\ldots\ldots\ldots\ldots\ldots\ldots\ldots\ldots\ldots \; (1)$$

when the fetus does not inherit the fragments with copy number variations from the mother, the parameter $\alpha$ is calculated as formula (2):

$$\alpha = \frac{(m-n)\cdot 2 + f \cdot n \cdot 2 + (1-f)\cdot n \cdot cn}{m \cdot 2} \quad\ldots\ldots\ldots\ldots\ldots \; (2)$$

wherein in formula (1) and formula (2), m represents the effective length of the chromosome in which the fragments with copy number variations occur, in the unit of Mb; and n represents the length of the fragments with copy number variations of the pregnant woman to be tested, in the unit of Mb; cn represents the copy number of the fragments with copy number variations found in the pregnant woman to be tested ;

wherein in formula (2), f represents the fraction of the cell-free fetal DNA existing in the peripheral blood cell-free DNA of the pregnant woman to be tested, and the fraction f of the cell-free fetal DNA is assumed to be less than 50%;and

$$x' = \frac{\hat{x}}{\alpha},$$

correcting the pre-correction coverage of each chromosome by using

wherein $x$ represents the pre-correction coverage of each chromosome and x' represents the corrected coverage of each chromosome.

In the prior art, the fragments with copy number variationss of the mother in the sequencing data will be removed directly without further consideration, however, the present invention is not the same as the prior art. The method of the present invention screens the fragment with copy number variations with certain length occurring on the chromosome of the mother, and the impact of the fragments with copy number variations on calculating the coverage of each chromosome is further removed while determining the aneuploidy of the chromosome, thereby obtaining a corrected coverage for each chromosome so as to achieve a more accurate result for chromosome aneuploidy according to the method of the present invention.

In above method of the present invention, the method for calculating the fraction f of cell-free fetal DNA contained in the peripheral blood cell-free DNA of the pregnant woman is a conventional calculation method in the art. For example, when the fetus is male, and when the fragments with copy number variations are on the X chromosome, the fraction of cell-free fetal DNA is calculated according to

$$f = 2\left(1 - \frac{\overline{N}_{23}}{\overline{N}}\right)$$

wherein $\dfrac{\overline{N}_{23}}{\overline{N}}$ represents the average number of the unique sequences in windows on the X chromosome to the average number of the unique sequences in all of the windows; and when fragments with copy number variations occur on chromosome 21, 18 or 13, the fraction of the cell-free fetal DNA is calculated as

$$f = 2(\frac{\overline{N}_i}{\overline{N}} - 1)\ :$$

wherein $\dfrac{\overline{N}_i}{\overline{N}}$ represents the ratio of the average number of the unique sequences in windows on the chromosome 21, 18 or 13 to the average number of the unique sequences in all of the windows. When the fetus is female, specific gene methylation detection for the peripheral blood cell-free DNA of the pregnant woman is needed. The principle is that certain genes have different forms of methylation in the DNA of the pregnant woman from the DNA of the fetus. For example, RASSF1A gene (on chromosome 3) from the fetus and the placental origins is highly methylated, however, RASSF1A gene from the mother herself is unmethylated. By treating the cff DNA by using methylation sensitive enzymes such as HhaI, BstUI (30U) and HpaII, the unmethylated gene will be digested and the methylated gene will not be digested, by which the content of fetal cff DNA can be detected through Q-PCR. The specific procedures are referenced in PLOS ONE 9: 71-7 (2014), Quantification of Cell-Free DNA in Normal and Complicated Pregnancies: Overcoming Biological and Technical Issues.

In the above-described method of the present invention, while calculating the pre-correction coverage of each chromosome, because the chromosome is segmented into windows for calculation, relatively robust chromosome coverage can be obtained. Thus, in a preferred embodiment of the present invention, the coverage statistics is calculated by segmenting all of the chromosomes in the sequencing data into windows with equal sizes so as to produce the pre-correction coverage of each chromosome.

In a more preferred embodiment of the present invention, during process of the calculation of coverage by segmenting into windows, the length of each window is 100 kb and the overlapping ratio between two adjacent windows is 50%. By controlling the length of each window as 100 kb and the ratio of overlap between the two adjacent windows as 50%, one cannot only obtain a relatively more robust chromosome coverage, but can also increase the accuracy for the detection of the fragments with copy number variations through the increased overlapping ratio between windows so as to increase the detection efficiency of the fragments with copy number variations of the pregnant woman.

In the above-described method of the present invention, based on the procedures of conventional methods for calculating the fragments with copy number variations, and according to the difference of the qualities of the sequencing data or accuracies of detections, it can be obtained by appropriately adjusting the condition met by the fragments with copy number variations. In a preferred embodiment of the present invention, calculating a $Z_{CNV}$ value of the number of unique sequences in each window of the pregnant woman and then locating chromosomal fragments with copy number variations of the pregnant woman to be tested on the basis of the magnitude of the $Z_{CNV}$ value further comprises the steps of:

counting the number of the unique sequences in each window according to the sequencing depth of each sequence in the sequencing data;
calculating the number of the unique sequences in each window according to the GC content and the mapping rate of each chromosome to obtain the pre-correction coverage of the number of the unique sequences in each window; and
normalizing the pre-correction coverage of the number of the unique sequences in each window to obtain the $Z_{CNV}$ value of the number of the unique sequences in each window and determine whether the pregnant woman to be tested has the chromosomal fragments with copy number variations on the basis of the magnitude of the $Z_{CNV}$ value;
if there is a fragment which is 300 kb or more in length in the sequencing data, and within the fragments which are 300 kb or more in length, the $Z_{CNV}$ values of the numbers of the unique sequences in 80% or more of the total windows are greater than or equal to 4 or less than or equal to -4, then the fragment which is 300 kb or more in length is determined to be the fragments with copy number variations from the pregnant woman to be tested.

In above step of normalizing the pre-correction coverage of the number of the unique sequences in each window to obtain the $Z_{CNV}$ value of the number of the unique sequences in each window, the normalizing treatment refers to performing (x-u)/sd(x-u) for the corrected value of the number of unique sequences in each window, wherein x is the corrected value, and u is the mean value of x, sd is the standard deviation. In above step of detecting the fragments with copy number variations of the pregnant woman, by setting the condition of "at least 300 kb, and $Z_{CNV}$ values in more than 80% of the total windows are greater than or equal to 4 or less than or equal to -4", a reliable copy number variation fragment of the pregnant woman can be detected by above detection steps of the present invention. By correcting the $Z_{CNV}$ value of the chromosome it occurs through the fragments with copy number variations, the false negative resulted by error detection of the fragments with copy number variations of the pregnant woman can be avoided.

In the above method of the present invention, for the step of performing a Z-test for each chromosome by using the corrected coverage of each chromosome to obtain the $Z_{aneu}$ value, the $Z_{aneu}$ value is calculated as:

$$Z_{aneu} = \frac{x' - \overline{x}}{s}$$

wherein $\overline{x}$ represents the pre-correction coverage obtained from a known negative sample population according to a LOESS algorithm; s represents the standard error of $(x'-\overline{x})$ in the negative sample population. Through the corrected $Z_{aneu}$ value calculated by above formula can indicate the chromosome aneuploidy more accurately, which will bring a more accurate result.

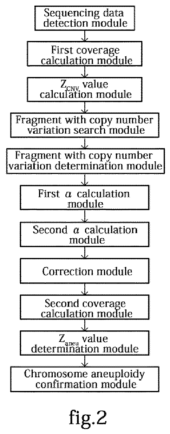

In another exemplary embodiment of the present invention, an apparatus for detecting chromosome aneuploidy is provided, as shown in Figure 2, comprising the following modules:

a sequencing data detection module: for high-throughput sequencing of the peripheral blood cell-free DNA from a pregnant woman to produce the sequencing data comprising all the chromosomes;

a first coverage calculation module: for calculating coverage statistics of all of the chromosomes with the sequencing data by segmenting chromosomes into windows so as to produce a pre-correction coverage for each chromosome;

a $Z_{CNV}$ value calculation module: for calculating the $Z_{CNV}$ value on the number of unique sequences in each window from the pregnant woman;

a fragments with copy number variations search module: for searching the fragment that is 300 kb or more in length in the sequencing data and which has the $Z_{CNV}$ values of the chromosome fragments greater than or equal to 4 or less than or equal to -4 in 80% or more of the total windows;

a fragments with copy number variations determination module: for determining a fragment in the sequencing data that is 300 kb or more in length and which has $Z_{CNV}$ values of the chromosome fragments greater than or equal to 4 or less than or equal to -4 in 80% or more of the total windows as the fragments with copy number variations of the pregnant woman;

a first $\alpha$ calculation module: for calculating a parameter $\alpha$ according to formula (1) in the case where the fetus inherits the fragments with copy number variations from the mother, wherein the parameter $\alpha$ represents the impact of the fragments with copy number variations of the pregnant woman on the pre-correction coverage of each chromosome; wherein in formula (1) m represents the effective length of the chromosome in which the fragments with copy number variations occur, in the unit of Mb; and n represents the length of the fragments with copy number variations of the pregnant woman, in the unit of Mb; cn represents the copy number of the fragments with copy number variations found in the pregnant woman;

$$\alpha = \frac{(m-n)\cdot 2 + n \cdot cn}{m \cdot 2} \quad \ldots\ldots\ldots\ldots\ldots\ldots\ldots\ldots\ldots\ldots\ldots \quad (1)$$

a second $\alpha$ calculation module: for calculating a parameter $\alpha$ according to formula (2) in the case where the fetus does not inherit the fragments with copy number variations from the mother, wherein the parameter $\alpha$ is calculated according to formula (2)

$$\alpha = \frac{(m-n)\cdot 2 + f \cdot n \cdot 2 + (1-f)\cdot n \cdot cn}{m \cdot 2} \quad \ldots\ldots\ldots\ldots\ldots \quad (2)$$

wherein in formula (2), m represents the effective length of the chromosome in which the fragments with copy number variations occurs, in the unit of Mb; and n represents the length of the fragments with copy number variations of the pregnant woman, in the unit of Mb; cn represents the copy number of the fragments with copy number variations found in the pregnant woman;, f represents the fraction of the cell-free fetal DNA existing in the peripheral blood cell-free DNA of the pregnant woman, and the fraction f of the cell-free fetal DNA is assumed to be less than 50%;

a correction module: for correcting the pre-correction coverage of each chromosome by using:

$$x' = \frac{\hat{x}}{\alpha}.$$

to produce the corrected coverage of each chromosome; wherein $\hat{x}$ represents the pre-correction coverage of each chromosome and $x'$ represents the corrected coverage of each chromosome;

a second coverage calculation module: for calculating the $Z_{aneu}$ value of each chromosome by using the corrected coverage of each chromosome;

$Z_{aneu}$ value determination module: for determining whether the absolute $Z_{aneu}$ value is greater than or equal to 3;

a chromosome aneuploidy confirmation module: for confirming the chromosome has aneuploidy in the case where the absolute $Z_{aneu}$ value is greater than or equal to 3.

In the above-described apparatus of the present invention, by adding a copy number variation fragment search module, a copy number variation fragment determination module and a correction module, and through screening a region that is at least 300 kb and has a $Z_{CNV}$ values greater than or equal to 4 or less than or equal to -4 in not less than 80% of the windows on the chromosome of the mother, the fragments with copy number variations of the pregnant woman can be detected by the apparatus of the present invention in a more reliable way. In addition, by correcting the Z-test value of the chromosome it occurs through the fragments with copy number variations, the false negative resulted by error detection of the fragments with copy number variations of the pregnant woman can be avoided. By correcting the impact of the fragments with copy number variations on the calculated coverage of each chromosome, the chromosome aneuploidy confirmation module of the present invention can confirm the chromosome aneuploidy in a more accurate way. In the correction module of above apparatus of the present invention, the fetal fraction in the calculation formula of parameter $\alpha$ is calculated by the conventional method in the art as described before, which will not be repeated here.

It should be noted that the above-described modules of the present invention can be operated as a part of the apparatus in a computing terminal, and the technical solutions achieved by the sequencing data detection module, the first coverage calculation module, the unique sequence calculation module, the fragments with copy number variations search module, the fragments with copy number variations determination module, the first $\alpha$ calculation module, the second $\alpha$ calculation module, the correction module, the second coverage calculation module and the chromosome aneuploidy confirmation module can be executed through using the operator provided by the computing terminal. It is clear that the computing terminal is the hardware apparatus and the operator is also the hardware for executing the program. In addition, the above mentioned sub-module of the present invention can run in a computing device such as the mobile terminal, computer terminal and the like, or can be stored as a part of the storage media.

In the above-described apparatus of the present invention, the first coverage calculation module may be obtained by appropriate adjustment according to the difference of sequencing data on the basis of the conventional computing module in the art. In a preferred embodiment of the present invention, the first coverage calculation module comprises:

a chromosome window segmentation sub-module: for segmenting all of the chromosomes in the sequencing data into windows with equal size;

a first coverage calculation sub-module: for calculating the coverage statistics in the form of windows with equal size to produce the pre-correction coverage of each chromosome.

Through the calculation in the form of segmented windows with equal size by the first coverage calculation module including the chromosome window segmentation sub-module and the first coverage calculation sub-module, a relatively more robust coverage can be obtained.

In a more preferred embodiment of the present invention, the length of each window in the chromosome window segmentation sub-module is 100 kb, and the overlapping ratio between two adjacent windows is 50%. The calculation module which performs the calculation by segmenting each window into the size of 100 kb is advantageous in obtaining a relatively more accurate coverage. In the other hand, by increasing the overlapping ratio between windows, the accuracy for the detection of the fragments with copy number variations can be increased so as to increase the detection efficiency of the fragments with copy number variations of the pregnant woman.

In the above-described apparatus of the present invention, a unique sequence calculation module may be obtained by using a conventional calculation module. In a preferred embodiment of the present invention, the unique sequence calculation module further comprises:

a unique sequence counting unit: for counting the number of the unique sequences in each window according to the sequencing depth of each sequence in the sequencing data;

a unique sequence coverage calculation unit: for calculating the number of the unique sequences in each window according to the GC content and the mapping rate of each chromosome to obtain the pre-correction coverage of the number of the unique sequences in each window; and

a unique sequence $Z_{CNV}$ value calculation unit: for normalizing the pre-correction coverage of the number of the unique sequences in each window to obtain the $Z_{CNV}$ value of the number of the unique sequences in each window.

In above unique sequence calculation module of the present invention, first, according to the sequencing depth of each sequence in the sequencing data, the number of the unique sequences in each window is counted by running the unique sequence counting unit, and then unique sequence coverage calculation unit is executed according to the GC content and the mapping rate of each chromosome to calculate the number of the unique sequence of each window to obtain the pre-correction coverage of the number of the unique sequences in each window, and then the unique sequence $Z_{CNV}$ value calculation unit is operated to normalize the pre-correction coverage of the number of the unique sequences in each window to obtain the $Z_{CNV}$ value of the number of the unique sequences in each window. Above units can be adjusted based on the conventional computing units in the art, which are the foundations and prerequisites for the searching of the fragments with copy number variations search module and as well as the confirmation of the chromosome aneuploidy confirmation module, which provide basis for accurately determining the fragments with copy number variations in the DNA of the mother in the sample to be tested.

In the above-described apparatus of the present invention, in the second coverage calculation module, the $Z_{aneu}$ value is calculated as:

$$Z_{aneu} = \frac{x' - \overline{x}}{s}$$

wherein $\overline{x}$ is the pre-correction coverage obtained from a known negative sample population according to a LOESS algorithm; s represents the standard error of $(x' - \overline{x})$ in the negative sample population. The corrected $Z_{aneu}$ value calculated by above formula can more accurately reflect the aneuploidy of the chromosome, making the detection result more accurate.

In yet another exemplary embodiment of the present invention, a kit for detecting chromosome aneuploidy is provided, the kit comprising:

the detection reagents and a detection device: for high-throughput sequencing of the peripheral blood cell-free DNA from a pregnant woman to be tested to produce the sequencing data containing all the chromosomes;

a first coverage calculation device: for calculating coverage statistics of all of the chromosomes with the sequencing data by segmenting the chromosomes into windows so as to produce a pre-correction coverage for each chromosome;

a $Z_{CNV}$ value calculation device: for performing a Z-test on the number of unique sequences in each window of the pregnant woman to be tested to obtain the $Z_{CNV}$ value;

a fragments with copy number variations search device: for searching the fragment in the sequencing data that is 300 kb or more in length and which has $Z_{CNV}$ values of the chromosome fragments greater than or equal to 4 or less than or equal to -4 in not less than 80% or more of the total windows;

a fragments with copy number variations determination device: for obtaining the fragments with copy number variations of the pregnant woman to be tested on the basis of the magnitude of the $Z_{CNV}$ value;

a first $\alpha$ calculation device: for calculating a parameter $\alpha$ according to the formula (1) in the case where the fetus inherits the fragments with copy number variations from the mother, wherein the parameter $\alpha$ represents the impact of the fragments with copy number variations of the pregnant woman to be tested on the pre-correction coverage of each chromosome;

$$\alpha = \frac{(m-n) \cdot 2 + n \cdot cn}{m \cdot 2} \quad \dots\dots\dots\dots\dots\dots\dots\dots\dots (1)$$

wherein m represents the effective length of the chromosome in which the fragments with copy number variations occurs, in the unit of Mb; and n represents the length of the fragments with copy number variations of the pregnant woman, in the unit of Mb; cn represents the copy number of the fragments with copy number variations found in the pregnant woman;

a second $\alpha$ calculation device: for calculating a parameter $\alpha$ according to formula (2) in the case where the fetus does not inherit the fragments with copy number variations from the mother, wherein the parameter α is calculated according to formula (2)

$$\alpha = \frac{(m-n)\cdot 2 + f\cdot n\cdot 2 + (1-f)\cdot n\cdot cn}{m\cdot 2} \qquad \ldots\ldots\ldots\ldots\ (2)$$

wherein m represents the effective length of the chromosome in which the fragments with copy number variations occurs, in the unit of Mb; and n represents the length of the fragments with copy number variations of the pregnant woman, in the unit of Mb; cn represents the copy number of the fragments with copy number variations found in the pregnant woman; f represents the fraction of the cell-free fetal DNA existing in the peripheral blood cell-free DNA of the pregnant woman, and the fraction f of the cell-free fetal DNA is assumed to be less than 50%;
a correction device: for correcting the pre-correction coverage of each chromosome by using:

$$x' = \frac{\hat{x}}{\alpha}.$$

to produce the corrected coverage of each chromosome; wherein $\hat{x}$ represents the pre-correction coverage of each chromosome and x' represents the corrected coverage of each chromosome;
a second coverage calculation device: for calculating the $Z_{aneu}$ value of each chromosome by using the corrected coverage of each chromosome;
$Z_{aneu}$ value determination device: for determining whether the absolute $Z_{aneu}$ value is greater than or equal to 3;
a chromosome aneuploidy confirmation device: for confirming the chromosome has aneuploidy in the case where the absolute $Z_{aneu}$ value is greater than or equal to 3.

In the kit of the present invention, by adding a fragments with copy number variations search device, a fragments withcopy number variations determination device and a correction device, and through screening a region that is at least 300 kb and which has $Z_{CNV}$ values greater than or equal to 4 or less than or equal to -4 in 80% or more of the total windows on the chromosome of the mother, the fragments with copy number variations of the pregnant woman can be detected by the kit of the present invention in a more reliable way. In addition, by correcting the $Z_{CNV}$ value of the chromosome it occurs through the fragments with copy number variations, the false negative resulted by error detection of the fragments with copy number variations of the pregnant woman can be avoided. By correcting the impact of the fragments with copy number variations on the calculated coverage of each chromosome, the chromosome aneuploidy confirmation device of the present invention can confirm the chromosome aneuploidy in a more accurate way. In the correction device of above kit of the present invention, the fetal fraction in the calculation formula of parameter α is calculated by the conventional method in the art as described before, which will not be repeated here.

It should be noted that the above-described devices of the present invention can be operated as a part of the apparatus in a computing terminal, and the technical solutions achieved by the sequencing data detection device, the first coverage calculation device, the unique sequence calculation device, the fragments with copy number variations search device, the fragments with copy number variations determination device, the first α calculation device, the second α calculation device, the correction device, the second coverage calculation device and the chromosome aneuploidy confirmation device can be executed through using the operator provided by the computing terminal. It is clear that the computing terminal is the hardware apparatus and the operator is also the hardware for executing the program. In addition, each above mentioned sub-device of the present invention can run in a computing device such as the mobile terminal, computer terminal and the like, or can be stored as a part of the storage media.

In the above-described kit of the present invention, the first coverage calculation device may be obtained by appropriate adjustment according to the difference of sequencing data on the basis of the conventional computing device in the art. In a preferred embodiment of the present invention, the first coverage calculation device includes
a chromosome window segmentation component: for segmenting all of the chromosomes in the sequencing data into windows with equal size;
a first coverage calculation component: for calculating the coverage statistics in the form of windows with equal size to produce the pre-correction coverage of each chromosome.

Through the calculation in the form of segmented windows with equal size by the first coverage calculation device including the chromosome window segmentation component and the first coverage calculation component, a relatively more robust coverage can be obtained.

In a more preferred embodiment of the present invention, the size of each window in the chromosome window segmentation component is 100 kb, and the overlapping ratio between two adjacent windows is 50%. The calculation device which performs the calculation by segmenting each window into the size of 100 kb is advantageous in obtaining a relatively more accurate coverage. In the other hand, by increasing the overlapping ratio between windows, the accuracy for the detection of the fragments with copy number variations can be increased so as to increase the detection efficiency of the fragments with copy number variations of the pregnant woman.

In the above-described kit of the present invention, a unique sequence calculation device may be obtained by using a conventional calculation device. In a preferred embodiment of the present invention, the sequence $Z_{CNV}$ value calculation device further includes:

a unique sequence counting component: for counting the number of the unique sequences in each window according to the sequencing depth of each sequence in the sequencing data;

a unique sequence overage calculation component: for calculating the number of the unique sequences in each window according to the GC content and the mapping rate of each chromosome to obtain the pre-correction coverage of the number of the unique sequences in each window; and

a unique sequence $Z_{CNV}$ value calculation component: for normalizing the pre-correction coverage of the number of the unique sequences in each window to obtain the $Z_{CNV}$ alue of the number of the unique sequences in each window.

In above unique sequence calculation device of the present invention, first, according to the sequencing depth of each sequence in the sequencing data, the number of the unique sequences in each window is counted by running the unique sequence counting unit, and then unique sequence coverage calculation unit is executed according to the GC content and the mapping rate of each chromosome to obtain the pre-correction coverage of the number of the unique sequences in each window, and then the unique sequence $Z_{CNV}$ value calculation sub-unit is operated to normalize the pre-correction coverage of the number of the unique sequences in each window to obtain the $Z_{CNV}$ value of the number of the unique sequences in each window. Above units can be adjusted based on the conventional computing units in the art, which are the foundations and prerequisites for the searching of the fragments with copy number variations search device and as well as the confirmation of the chromosome aneuploidy confirmation device, which provide basis for accurately determining the fragments with copy number variations in the DNA of the mother of the to be tested samples.

In the above-described kit of the present invention, in the second coverage calculation device, the $Z_{aneu}$ value is calculated as:

$$Z_{aneu} = \frac{x' - \overline{x}}{s}$$

wherein $\overline{x}$ is the pre-correction coverage obtained by the known negative sample population according to a LOESS algorithm; s represents the standard error of $(x' - \overline{x})$ in the negative sample population. The corrected $Z_{aneu}$ value calculated by above formula can more accurately reflect the aneuploidy of the chromosome, making the detection result more accurate.

The beneficial impacts of the present invention will be further described below in combination with specific examples.

EXAMPLES

Example 1

In order to test the impact of the correction of the fragments with copy number variations of the pregnant woman on the correction of the chromosome aneuploidy, this example generated a set of simulated data for a to be tested pregnant woman based on the Poisson distribution. In this simulated data, a quantitative copy number of abnormal fragments were added to chromosome 13, 18 and 21, respectively, and the sizes of those copy number variation fragments are from 0.5Mb to 5Mb, wherein the step length is 0.25Mb. Then 3 different fraction s (5%, 10%, 15%) of DNA from normal people were mixed into the simulated data containing the fragments with copy number variations. The whole process is to mimic the impact of the size of different copy number variation fragments on the coverage of chromosome 13, 18 and 21 under different fetal fraction s, and to further test the corrected impact of the fragments with copy number variations of the pregnant woman on the detection of the chromosome aneuploidy. All of the calculations were performed under the assumption that the fetus does not inherent the fragments with copy number variations of the pregnant woman.

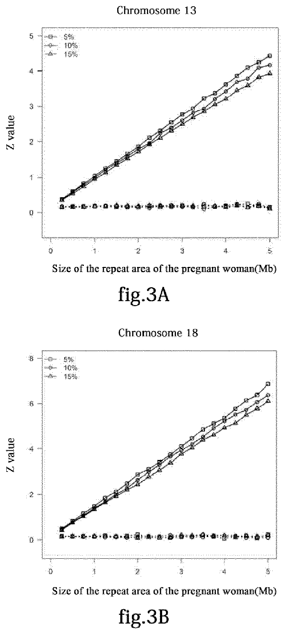

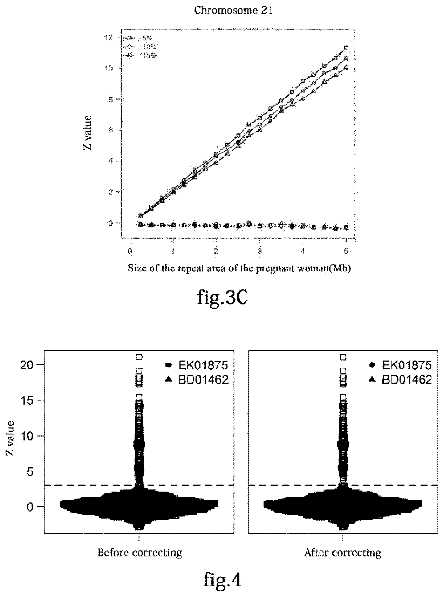

The results of the test are shown in Figures 3A, 3B and 3C. In above three figures, the abscissas represents the sizes of the fragments with copy number variations of the pregnant woman where the sample came from, and the ordinates represents the Z values of the chromosomes of this sample. The solid line in the figure shows the Z values of the chromosomes before correction, and the dotted line shows the Z values calculated by the coverage of the chromosomes after the correction through the fragments with copy number variations of the pregnant woman, i.e. $Z_{aneu}$ value. Square, round and triangular indicates 5%, 10% and 15% fetal fraction s in the samples, respectively.

As can be clearly seen from Figure 3A, 3B and 3C, when the Z value was calculated directly with the chromosome coverage, the Z value of the sample increased as the size of the fragments with copy number variations of the pregnant woman increased. In the case of chromosome 21, for example, at 10% fetal fraction, if there is a 3Mb repeat on chromosome 21 of the pregnant woman, even the fetus does not have 21 trisomy syndrome, the Z value calculated by the previous coverage will be more than 3, which will be determined as a positive. However, as shown by the dotted line, the Z value calculated by the corrected coverage through the method of the present invention, i.e. $Z_{aneu}$ value, were all around the baseline 0, which means that the method of the present invention for detecting the chromosome aneuploidy corrected by utilizing the fragments with copy number variations of the pregnant woman is extremely effective.

In order to further verify the effects of the method, the apparatus and the kit provided on the detection of the chromosome aneuploidy in real patients' samples, the following samples from the patients were detected through the method, the apparatus and the kit of the present invention as further described in Example 2 and Example 3.

Example 2

High-throughput sequencing was performed for peripheral blood cell-free DNA from 6615 pregnant women to be tested to produce the sequencing data comprising all of the chromosomes in the samples.

The number of the unique sequences in each window was counted according to the depth of sequencing for each of the sequences in the sequencing data; and the number of the unique sequences in each window was corrected according to the GC content and the mapping rate of each chromosome to produce the corrected coverage of the number of the unique sequences in each window; and the pre-correction coverage of the number of the unique sequences in each window was normalized to produce the $Z_{CNV}$ value of the number of the unique sequences in each window and to determine whether the pregnant woman possesses the fragments with copy number variations on the basis of the magnitude of the $Z_{CNV}$ value; when there is a fragment 300 kb or more in the sequencing data, and for the fragment 300 kb or more, the $Z_{CNV}$ value of the number of the unique sequences in 80% or more of the windows is greater than or equal to 4 or less than or equal to -4, the fragment 300 kb or more is determined to be the fragments with copy number variations of the pregnant woman.

By utilizing the impact of the fragments with copy number variations of the pregnant woman on the pre-correction coverage of each chromosome, i.e. parameter $\alpha$, the pre-correction coverage was correct by using

$$x^{'} = \frac{\hat{x}}{\alpha}.$$

to produce the corrected coverage of each chromosome; wherein $\hat{x}$ represents the pre-correction coverage of each chromosome and x' represents the corrected coverage of each chromosome, and impact of the fragments with copy number variations of the pregnant woman on the pre-correction coverage of each chromosome parameter $\alpha$ was calculated by formula (1) or (2);

The $Z_{aneu}$ value was calculated by using the corrected coverage of each chromosome according to formula:

$$Z_{aneu} = \frac{x^{'} - \overline{x}}{s}$$

and the aneuploidy of the chromosome was determined based on whether the absolute value of $Z_{aneu}$ is greater than or equal to 3; wherein when the absolute value of $Z_{aneu}$ is greater than or equal to 3, the chromosome has aneuploidy, and when the absolute value of $Z_{aneu}$ is less than or equal to 3, the chromosome does not have aneuploidy.

Through above detection method of the present invention, it was found that copy number variation fragments of the pregnant woman exist on chromosome 21 of sample EK01875 and BD01462, and the positive results of those two samples were corrected into negative results as shown in Fig. 4 in detail.

The left panel of Fig. 4 (see the figure with color) shows the statistical Z value of chromosome 21 of all the samples detected by the detection methods in the art. As can be seen, the Z values of the negative samples are almost all less than 3 which are close to a normal distribution. The round in the figure indicates sample EK01875 with a Z value of 4.66. The triangle indicates sample BD01462 with a Z value of 3.87.

The right panel of Fig. 4 shows the statistical Z value of chromosome 21 obtained by the detection method of the present invention, wherein the sample EK01875 has $Z_{aneu}$ = 2.36, and the sample BD01462 has $Z_{aneu}$ = 1.83.

Example 3

Above sample (EK01875, 29 years old pregnant woman at about 18w pregnancy) was detected by the detection apparatus of the present invention for chromosome aneuploidy, wherein the apparatus includes:

a sequencing data detecting module: for high-throughput sequencing the peripheral blood cell-free DNA of a pregnant woman to produce sequencing data comprising all the chromosomes;
a first coverage calculation module: for calculating a coverage statistics of all chromosomes in the sequencing data by segmenting into windows so as to produce a pre-correction coverage for each chromosome;
a $Z_{CNV}$ value calculation module: for calculating the $Z_{CNV}$ value on the number of unique sequences in each of the windows of the pregnant woman;
a fragments with copy number variations search module: for searching the fragment in the sequencing data that is 300 kb or more e in length in the sequencing data and which has the $Z_{CNV}$ values of the chromosome fragments greater than or equal to 4 or less than or equal to -4 in 80% or more of the total windows;
a fragments with copy number variations determination module: for determining a fragment in the sequencing data that is 300 kb or more and which has $Z_{CNV}$ values of the chromosome fragments greater than or equal to 4 or less than or equal to -4 in 80% or more of the total windows as the fragments with copy number variations of the pregnant woman;
a first $\alpha$ calculating module: for calculating the parameter $\alpha$ according to the formula (1) in the case where the fetus inherits the fragments with copy number variations from the mother;
a second $\alpha$ calculating module: for calculating the parameter $\alpha$ according to the formula (2) in the case where the fetus does not inherit the fragments with copy number variations from the mother;
a correcting module: for correcting the pre-correction coverage of each chromosome by using

$$x^{'} = \frac{\hat{x}}{\alpha}.$$

to produce the corrected coverage of each chromosome;
a second coverage calculating module: for calculating the $Z_{aneu}$ value of each chromosome by using the corrected coverage of each chromosome;
$Z_{aneu}$ value determination module: for determining whether the $Z_{aneu}$ value is greater than or equal to 3;
a chromosome aneuploidy confirming module: for confirming the chromosome has aneuploidy in the case where the $Z_{aneu}$ value is greater than or equal to 3.

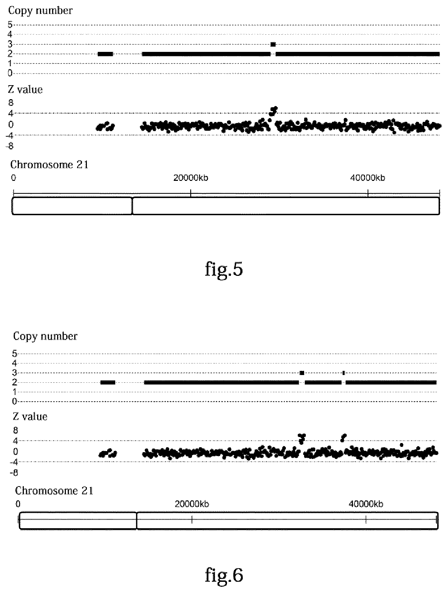

After analyzing the detection of chromosome aneuploidy by using above apparatus of the present invention, an 850 kb repeat was found on chromosome 21 of the pregnant woman. As seen in Fig. 5, the regions with repeated copies are 21q22.11 (32361194bp~32861193bp) of 500 kb and 21q22.12 (37261194bp~37611193bp) of 350 kb, respectively, and their copy numbers are both 3.

Then, the result of the fragments with copy number variations of the pregnant woman was further verified by the Affymetrix CytoScan 750k SNP chip in the art. Similarly, repeats were detected in regions of 21q22.11 (32399114bp~32811202bp) and 21q22.12 (37292432bp~37602701bp), and the copy numbers are both 3.

It can be seen that the positions detected by the chip are almost 100% identical to the positions detected by the apparatus of the present invention. According to the apparatus of the present invention, the impact of the fragments with copy number variations of the pregnant woman on the calculation of the coverage of the chromosome, i.e. parameter $\alpha$, was 1.012, which corrected the Z value characterizing the aneuploidy of the chromosome from 4.66 to 2.36, thereby the result is corrected into negative.

Example 4

Above sample (BD01462, 24 years old pregnant woman at about 24w pregnancy) was detected by the kit of the present invention for chromosome aneuploidy, wherein the kit comprises:

the detecting reagents and a detecting device: for high-throughput sequencing the peripheral blood cell-free DNA of a pregnant woman to be tested to produce the sequencing data containing all chromosomes;

a first coverage calculation device: for calculating coverage statistics for all of the chromosomes in the sequencing data by segmenting into windows so as to produce a pre-correction coverage for each chromosome;

a unique sequence calculation device: for calculating the $Z_{CNV}$ value of the number of unique sequences in each window of the to be tested pregnant woman;

a fragments with copy number variations search device: for searching the fragment in the sequencing data that is 300 kb or more and which has the $Z_{CNV}$ values of the chromosome fragments greater than or equal to 4 or less than or equal to -4 in 80% or more of the total windows;

a fragments with copy number variations determination device: for obtaining the fragments with copy number variations of the pregnant woman to be tested on the basis of the magnitude of the $Z_{CNV}$ value;

a first $\alpha$ calculation device: for calculating the parameter $\alpha$ according to the formula (1) in the case where the fetus inherits the fragments with copy number variations from the mother;

a second $\alpha$ calculation device: for calculating the parameter $\alpha$ according to the formula (2) in the case where the fetus does not inherit the fragments with copy number variations from the mother;

a correcting device: for correcting the pre-correction coverage of each chromosome by using

$$x^{'} = \frac{\hat{x}}{\alpha}.$$

to produce the corrected coverage of each chromosome;

a second coverage calculation device: for calculating the $Z_{aneu}$ value of each chromosome by using the corrected coverage of each chromosome;

$Z_{aneu}$ value determination device: for determining whether the $Z_{aneu}$ value is greater than or equal to 3;

a chromosome aneuploidy confirming device: for confirming the chromosome has aneuploidy in the case where the $Z_{aneu}$ value is greater than or equal to 3.

After analyzing the detection by above kit of the present invention, as shown in Fig. 6, a total of 700 kb repeat was found on chromosome 21 of the pregnant woman in region 21q23.1 (28911194bp~29611930), and the copy number is 3.

Similarly, 21q21.3 (28973792bp~29542400) repeat was found by using Affymetrix CytoScan 750k SNP chip.

Although the detected copy number is 4 which is slightly different from that of the present invention, the position in the result is almost 100% identical to that detected by the kit of the present invention, showing the accuracy of the detection method of the present invention. According to the kit of the present invention, the impact of the fragments with copy number variations of the pregnant woman on the coverage of the chromosome, i.e. parameter $\alpha$, was 1.009, which corrected the Z value characterizing the aneuploidy of the chromosome from 3.87 to 1.83, thereby correcting the result into negative.

As can be seen from above description, above examples of the present invention have achieved the following technical effects: when considering the influence of the fragments with copy number variations of the pregnant woman herself on the calculation of chromosome aneuploidy, the idea of removing the fragments with copy number variations of the mother from the sequencing data is abandoned, and the effect of the fragments with copy number variations with the certain size of the mother on calculating the chromosome aneuploidy is inventively represented by parameter $\alpha$, which is further used to correct the coverage of each chromosome so as to decrease the influence of the fragments with copy number variations on the determination of the chromosome aneuploidy. The presence of the fragments with copy number variations is not ignored, resulting in a more accurate result for chromosome aneuploidy detected by the method of the present invention.

The method, apparatus, or kit of the present invention provides a novel detection manner for NIPT of fetus chromosome aneuploidy without any interference from the fragments with copy number variations of the pregnant woman, which improves the accuracy of detection and is suitable for large-scale use.

It will be apparent to those skilled in the art that some of the modules, elements, or steps of the present invention described above may be implemented by general computing apparatus, and they can be integrated into one computing apparatus or distributed into a net composed of multiple computing apparatus. Optionally, they can be achieved by program code implementable by the computing apparatus so that they can be stored in a storage apparatus and executed by the computing apparatus. Or multiple modules or step among those can be made into individual integrated circuit modules. In this way, the present invention will not be limited by any particular hardware or software.

**Claims**

1. A method for detecting chromosome aneuploidy, which is **characterized in that** the method includes the following steps of:

   high-throughput sequencing of the peripheral blood cell-free DNA from a pregnant woman to be tested to produce sequencing data comprising all of the chromosomes;
   calculating coverage statistics for all of the chromosomes with the sequencing data by segmenting the chromosomes into windows so as to produce a pre-correction coverage of each chromosome;
   performing a Z-test on the number of unique sequences in each window of the pregnant woman to produce a $Z_{CNV}$ value and then locating chromosomal fragments with copy number variations of the pregnant woman on the basis of the magnitude of the $Z_{CNV}$ value; wherein the chromosomal fragments with copy number variations of the pregnant woman are 300 kb or more in length and have $Z_{CNV}$ values of the chromosome fragments greater than or equal to 4 or less than or equal to -4 in at least 80% of the total windows within the fragments which are 300 kb or more in length,
   correcting the pre-correction coverage of each chromosome by utilizing the impact of the fragments with copy number variations of the pregnant woman on the pre-correction coverage of each chromosome to produce the corrected coverage of each chromosome; and
   performing a Z-test for each chromosome by using the corrected coverage of each chromosome to obtain a $Z_{aneu}$ value, and determining chromosome aneuploidy based on whether the absolute value of $Z_{aneu}$ is greater than or equal to 3; wherein if the absolute value of $Z_{aneu}$ is greater than or equal to 3, chromosome aneuploidy is determined;
   wherein the impact of the fragments with copy number variations of the pregnant woman to be tested on the pre-correction coverage of each chromosome is represented by a parameter $\alpha$,
   when the fetus inherits the fragments with copy number variations from the mother, the parameter $\alpha$ is calculated as formula (1):

$$\alpha = \frac{(m-n)\cdot 2 + n \cdot cn}{m \cdot 2} \quad\dots\dots\dots\dots\dots\dots\dots\dots\dots\dots\quad (1)$$

   when the fetus does not inherit the fragments with copy number variations from the mother, the parameter $\alpha$ is calculated as formula (2):

$$\alpha = \frac{(m-n)\cdot 2 + f \cdot n \cdot 2 + (1-f)\cdot n \cdot cn}{m \cdot 2} \quad\dots\dots\dots\dots\quad (2)$$

   wherein in formula (1) and formula (2), m represents the effective length of the chromosome in which the fragments with copy number variations occur, in the unit of Mb; and n represents the length of the fragments with copy number variations of the pregnant woman to be tested, in the unit of Mb; cn represents the copy number of the fragments with copy number variations found in the pregnant woman to be tested;
   wherein in formula (2), f represents the fraction of the cell-free fetal DNA existing in the peripheral blood cell-free DNA of the pregnant woman to be tested, and the fraction f of the cell-free fetal DNA is assumed to be less than 50%;
   correcting the pre-correction coverage of each chromosome by using

$$x' = \frac{\hat{x}}{\alpha},$$

   wherein $\hat{x}$ represents the pre-correction coverage of each chromosome and x' represents the corrected coverage of each chromosome.

2. The method according to claim 1, wherein the coverage statistics is calculated by segmenting all of the chromosomes in the sequencing data into windows with equal sizes so as to produce the pre-correction coverage of each chromosome.

3. The method according to claim 2, wherein the length of each window is 100 kb and the overlapping ratio between two adjacent windows is 50%.

4. The method according to claim 1, wherein the step of performing a Z-test on the number of unique sequences in each window of the pregnant woman to be tested to produce the $Z_{CNV}$ value and then locating chromosomal fragments with copy number variations of the pregnant woman to be tested on the basis of the magnitude of the $Z_{CNV}$ value further includes the steps of:

counting the number of the unique sequences in each window according to the sequencing depth of each sequence in the sequencing data;
calculating the number of the unique sequences in each window according to the GC content and the mapping rate of each chromosome to obtain the pre-correction coverage of the number of the unique sequences in each window; and
normalizing the pre-correction coverage of the number of the unique sequences in each window to obtain the $Z_{CNV}$ value of the number of the unique sequences in each window and determining whether the pregnant woman to be tested has a chromosomal fragment with the copy number variation on the basis of the magnitude of the $Z_{CNV}$ value;
if there are fragments which are 300 kb or more in length in the sequencing data, and within the fragments which are 300 kb or more in length, the $Z_{CNV}$ values of the numbers of the unique sequences in at least 80% of the total windows are greater than or equal to 4 or less than or equal to -4, then the fragments which are 300 kb or more in length are determined to be the fragments with copy number variations of the pregnant woman to be tested.

5. The method according to claim 1, wherein for the step of performing a Z-test for each chromosome by using the corrected coverage of each chromosome to obtain the $Z_{aneu}$ value, the $Z_{aneu}$ value is calculated as:

$$Z_{aneu} = \frac{x' - \overline{x}}{s}$$

wherein $\overline{x}$ represents the pre-correction coverage obtained from a known negative sample population according to a LOESS algorithm; s represents the standard error of $(x' - \overline{x})$ in the negative sample population.

6. An apparatus for detecting chromosome aneuploidy, which is **characterized in that** the apparatus includes the following modules:

a sequencing data detection module: for high-throughput sequencing of the peripheral blood cell-free DNA from a pregnant woman to be tested to produce the sequencing data comprising all of the chromosomes;
a first coverage calculation module: for calculating coverage statistics of all of the chromosomes with the sequencing data by segmenting the chromosomes into windows so as to produce a pre-correction coverage of each chromosome;
a $Z_{CNV}$ value calculation module: for calculating the $Z_{CNV}$ value on the number of unique sequences in each window of the pregnant woman;
a fragments with copy number variations search module: for searching the fragment that is 300 kb or more in length in the sequencing data and which has the $Z_{CNV}$ values of the chromosome fragments greater than or equal to 4 or less than or equal to -4 in at least 80% of the total windows;
a fragments with copy number variations determination module: for determining fragments in the sequencing data that are 300 kb or more in length and which have $Z_{CNV}$ values of the chromosome fragments greater than or equal to 4 or less than or equal to -4 in at least 80% of the total windows as the fragments with copy number variations of the pregnant woman;
a first $\alpha$ calculation module: for calculating a parameter $\alpha$ according to formula (1) in the case where the fetus inherits the fragments with copy number variations from the mother, wherein the parameter $\alpha$ represents the impact of the fragments with copy number variations of the pregnant woman on the pre-correction coverage of each chromosome;

$$\alpha = \frac{(m-n)\cdot 2 + n\cdot cn}{m\cdot 2} \quad \dots\dots\dots\dots\dots\dots\dots\dots\dots\dots \quad (1)$$

wherein in formula (1), m represents the effective length of the chromosome in which the fragments with copy number variations occur, in the unit of Mb; and n represents the length of the fragments with copy number variations of the pregnant woman, in the unit of Mb; cn represents the copy number of the fragments with copy number variations found in the pregnant woman;

a second $\alpha$ calculation module: for calculating a parameter $\alpha$ according to formula (2) in the case where the fetus does not inherit the fragments with copy number variations from the mother, wherein the parameter $\alpha$ is calculated according to formula (2)

$$\alpha = \frac{(m-n)\cdot 2 + f\cdot n\cdot 2 + (1-f)\cdot n\cdot cn}{m\cdot 2} \quad \dots\dots\dots\dots\dots \quad (2)$$

wherein in formula (2), m represents the effective length of the chromosome in which the fragments with copy number variations occur, in the unit of Mb; and n represents the length of the fragments with copy number variations of the pregnant woman, in the unit of Mb; cn represents the copy number of the fragments with copy number variations found in the pregnant woman; f represents the fraction of the cell-free fetal DNA existing in the peripheral blood cell-free DNA of the pregnant woman, and the fraction f of the cell-free fetal DNA is assumed to be less than 50%;

a correction module: for correcting the pre-correction coverage of each chromosome by using: $x' = \dfrac{\hat{x}}{\alpha}$ to produce the corrected coverage of each chromosome; wherein $\hat{x}$ represents the pre-correction coverage of each chromosome and x' represents the corrected coverage of each chromosome;

a second coverage calculation module: for calculating the $Z_{aneu}$ value of each chromosome by using the corrected coverage of each chromosome;

$Z_{aneu}$ value determination module: for determining, whether the absolute $Z_{aneu}$ value is greater than or equal to 3;

a chromosome aneuploidy confirmation module: for confirming the chromosome is aneuploid in the case where the absolute $Z_{aneu}$ value is greater than or equal to 3.

7. The apparatus according to claim 6, wherein the first coverage calculation module includes:

   a chromosome window segmentation sub-module: for segmenting all of the chromosomes in the sequencing data into windows with equal size;

   a first coverage calculation sub-module: for calculating coverage statistics in the form of windows with equal size to produce the pre-correction coverage of each chromosome.

8. The apparatus according to claim 7, wherein the length of each window in the chromosome window segmentation sub-module is 100 kb, and the overlapping ratio between two adjacent windows is 50%.

9. The apparatus according to claim 6, wherein the $Z_{CNV}$ value calculation module includes:

   a unique sequence counting unit: for counting the number of the unique sequences in each window according to the sequencing depth of each sequence in the sequencing data;

   a unique sequence coverage calculation unit: for calculating the number of the unique sequences in each window according to the GC content and the mapping rate of each chromosome to obtain the pre-correction coverage of the number of the unique sequences in each window; and

   a unique sequence $Z_{CNV}$ value calculation unit: for normalizing the pre-correction coverage of the number of the unique sequences in each window to obtain the $Z_{CNV}$ value of the number of the unique sequences in each window.

10. The apparatus according to claim 6, wherein in the second coverage calculation module, the $Z_{aneu}$ value is calculated

as:

$$Z_{aneu} = \frac{x' - \bar{x}}{s}$$

wherein $\bar{x}$ is the pre-correction coverage obtained from a known negative sample population according to a LOESS algorithm; s represents the standard error of $(x' - \bar{x})$ in the negative sample population.

11. A kit for detecting chromosome aneuploidy, which is **characterized in that** the kit includes:

the detection reagents and a detection device: for high-throughput sequencing of the peripheral blood cell-free DNA from a pregnant woman to be tested to produce the sequencing data containing all the chromosomes;
a first coverage calculation device: for calculating coverage statistics of all of the chromosomes with the sequencing data by segmenting the chromosomes into windows so as to produce a pre-correction coverage of each chromosome;
a $Z_{CNV}$ value calculation device: for performing a Z-test on the number of unique sequences in each window of the pregnant woman to be tested to obtain the $Z_{CNV}$ value;
a fragments with copy number variations search device: for searching the fragments in the sequencing data that are 300 kb or more in length and which have $Z_{CNV}$ values of the chromosome fragments greater than or equal to 4 or less than or equal to -4 in at least 80% of the total windows;
a fragments with copy number variations determination device: for obtaining the fragments with copy number variations of the pregnant woman to be tested on the basis of the magnitude of the $Z_{CNV}$ value;
a first $\alpha$ calculation device: for calculating a parameter $\alpha$ according to formula (1) in the case where the fetus inherits the fragments with copy number variations from the mother, wherein the parameter $\alpha$ represents the impact of the fragments with copy number variations of the pregnant woman to be tested on the pre-correction coverage of each chromosome;

$$\alpha = \frac{(m-n) \cdot 2 + n \cdot cn}{m \cdot 2} \quad \dots\dots\dots\dots\dots\dots\dots\dots\dots\dots \ (1)$$

wherein m represents the effective length of the chromosome in which the fragments with copy number variations occur, in the unit of Mb; and n represents the length of the fragments with copy number variations of the pregnant woman to be tested, in the unit of Mb; cn represents the copy number of the fragments with copy number variations found in the pregnant woman;
a second $\alpha$ calculation device: for calculating a parameter $\alpha$ according to formula (2) in the case where the fetus does not inherit the fragments with copy number variations from the mother, wherein the parameter $\alpha$ is calculated according to formula (2)

$$\alpha = \frac{(m-n) \cdot 2 + f \cdot n \cdot 2 + (1-f) \cdot n \cdot cn}{m \cdot 2} \quad \dots\dots\dots\dots\dots \ (2)$$

wherein m represents the effective length of the chromosome in which the fragments with copy number variations occur, in the unit of Mb; and n represents the length of the fragments with copy number variations of the pregnant woman, in the unit of Mb; cn represents the copy number of the fragments with copy number variations found in the pregnant woman; f represents the fraction of the cell-free fetal DNA existing in the peripheral blood cell-free DNA of the pregnant woman, and the fraction f of the cell-free fetal DNA is assumed to be less than 50%;
a correction device: for correcting the pre-correction coverage of each chromosome by using:

$$x' = \frac{\hat{x}}{\alpha}$$

to produce the corrected coverage of each chromosome; wherein $\hat{x}$ represents the pre-correction coverage of each chromosome and x' represents the corrected coverage of each chromosome;

a second coverage calculation device: for calculating the $Z_{aneu}$ value of each chromosome by using the corrected coverage of each chromosome;

$Z_{aneu}$ value determination device: for determining whether the absolute $Z_{aneu}$ value is greater than or equal to 3;

a chromosome aneuploidy confirmation device: for confirming chromosome aneuploidy in the case where the absolute $Z_{aneu}$ value is greater than or equal to 3.

12. The kit according to claim 11, wherein the first coverage calculation device includes:

a chromosome window segmentation component: for segmenting all of the chromosomes in the sequencing data into windows with equal size;

a first coverage calculation component: for calculating the coverage statistics in the form of windows with equal size to produce the pre-correction coverage of each chromosome.

13. The kit according to claim 12, wherein the length of each window in the chromosome window segmentation component is 100 kb, and the overlapping ratio between two adjacent windows is 50%.

14. The kit according to claim 11, wherein the $Z_{CNV}$ value calculation device includes:

a unique sequence counting component: for counting the number of the unique sequences in each window according to the sequencing depth of each sequence in the sequencing data;

a unique sequence coverage calculation component: for calculating the number of the unique sequences in each window according to the GC content and the mapping rate of each chromosome to obtain the pre-correction coverage of the number of the unique sequences in each window; and

a unique sequence $Z_{CNV}$ value calculation component: for normalizing the pre-correction coverage of the number of the unique sequences in each window to obtain the $Z_{CNV}$ value of the number of the unique sequences in each window.

15. The kit according to claim 11, wherein in the second coverage calculation device, the $Z_{aneu}$ value is calculated as:

$$Z_{aneu} = \frac{x' - \overline{x}}{s}$$

wherein $\overline{x}$ is the pre-correction coverage obtained from a known negative sample population according to a LOESS algorithm; s represents the standard error of $(x' - \overline{x})$ in the negative sample population.

**Patentansprüche**

1. Verfahren zur Detektion von Chromosom-Aneuploidie, das dadurch charakterisiert ist, dass das Verfahren die folgenden Schritte beinhaltet:

Hochdurchsatz-Sequenzieren der zellfreien DNA aus peripherem Blut einer zu testenden schwangeren Frau, um Sequenzierungsdaten, die alle der Chromosomen umfassen, zu produzieren;

Berechnen einer Reichweitestatistik für alle der Chromosomen anhand der Sequenzierungsdaten durch Segmentieren der Chromosomen in Fenster um eine Vor-Korrektur-Reichweite jedes Chromosoms zu produzieren;

Durchführen eines Z-Tests für die Anzahl an einzigartigen Sequenzen in jedem Fenster der schwangeren Frau um einen $Z_{CNV}$-Wert zu produzieren, und dann Orten chromosomaler Fragmente mit Kopiezahlvariationen der schwangeren Frau auf Basis der Größenordnung des $Z_{CNV}$-Wertes; wobei die chromosomalen Fragmente mit Kopiezahlvariationen der schwangeren Frau 300 kb oder mehr lang sind und in mindestens 80% der gesamten Fenster innerhalb der Fragmente, die 300kb oder mehr lang sind, $Z_{CNV}$- Werte der Chromosomfragmente von größer als oder gleich 4 oder weniger als oder gleich -4 haben,

Korrigieren der Vor-Korrektur-Reichweite jedes Chromosoms durch Verwendung der Auswirkung der Fragmente mit Kopiezahlvariationen der schwangeren Frau auf die Vor-Korrektur-Reichweite jedes Chromosoms um die korrigierte Reichweite für jedes Chromosom zu produzieren; und

Durchführen eines Z-Tests für jedes Chromosom durch Verwendung der korrigierten Reichweite jedes Chromosoms um einen $Z_{aneu}$-Wert zu erhalten, und Feststellen von Chromosom-Aneuploidie basierend darauf, ob der absolute Wert von $Z_{aneu}$ größer als oder gleich 3 ist; wobei Chromosom-Aneuploidie festgestellt wird, wenn der absolute Wert von $Z_{aneu}$ größer als oder gleich 3 ist;

wobei die Auswirkung der Fragmente mit Kopiezahlvariationen der zu testenden schwangeren Frau auf die Vor-Korrektur-Reichweite jedes Chromosoms durch einen Parameter a repräsentiert ist,

wenn der Fötus die Fragmente mit Kopiezahlvariationen von der Mutter erbt, wird der Parameter a berechnet als Formel (1):

$$\alpha = \frac{(m-n)\cdot 2 + n\cdot cn}{m\cdot 2} \quad\dots\dots\dots\dots\dots\dots\dots\dots\dots\dots\dots\dots \text{（1）}$$

wenn der Fötus die Fragmente mit Kopiezahlvariationen nicht von der Mutter erbt, wird der Parameter a berechnet als Formel (2):

$$\alpha = \frac{(m-n)\cdot 2 + f\cdot n\cdot 2 + (1-f)\cdot n\cdot cn}{m\cdot 2} \quad\dots\dots\dots\dots\dots \text{（2）}$$

wobei in Formel (1) und Formel (2) m die effektive Länge des Chromosoms, in dem die Fragmente mit Kopiezahlvariationen vorkommen, repräsentiert, in der Einheit Mb; und n die Länge der Fragmente mit Kopiezahlvariationen der zu testenden schwangeren Frau repräsentiert, in der Einheit Mb; cn die Kopiezahl der Fragmente mit Kopiezahlvariationen, die in der zu testenden schwangeren Frau gefunden wird, repräsentiert;

wobei in Formel (2), f den Bruchteil der zellfreien fötalen DNA, die in der zellfreien DNA aus dem peripheren Blut der zu testenden schwangeren Frau existiert, repräsentiert, und der Bruchteil f der zellfreien fötalen DNA als weniger als 50% angenommen wird;

$$x' = \frac{\hat{x}}{\alpha},$$

Korrigieren der Vor-Korrektur-Reichweite jedes Chromosoms durch Verwendung von

wobei $\hat{x}$ die Vor-Korrektur-Reichweite jedes Chromosoms repräsentiert, und x' die korrigierte Reichweite jedes Chromosoms repräsentiert.

2. Verfahren nach Anspruch 1, wobei die Reichweitestatistik durch Segmentieren aller der Chromosomen in den Sequenzierungsdaten in Fenster mit gleichen Größen, um die Vor-Korrektur-Reichweite jedes Chromosoms zu produzieren, berechnet wird.

3. Verfahren nach Anspruch 2, wobei die Länge jedes Fensters 100 kb ist und das Überlappungsverhältnis zwischen zwei nebeneinanderliegenden Fenstern 50% ist.

4. Verfahren nach Anspruch 1, wobei der Schritt des Durchführens eines Z-Tests für die Anzahl an einzigartigen Sequenzen in jedem Fenster der zu testenden schwangeren Frau um den $Z_{CNV}$- Wert zu produzieren, und dann Orten chromosomaler Fragmente mit Kopiezahlvariationen der zu testenden schwangeren Frau auf Basis der Größenordnung des $Z_{CNV}$-Wertes weiterhin die Schritte:

Zählen der Anzahl der einzigartigen Sequenzen in jedem Fenster gemäß der Sequenzierungstiefe jeder Sequenz in den Sequenzierungsdaten;

Berechnen der Anzahl der einzigartigen Sequenzen in jedem Fenster gemäß dem GC-Gehalt und der Zuordnungsrate jedes Chromosoms, um die Vor-Korrektur-Reichweite der Anzahl der einzigartigen Sequenzen in jedem Fenster zu erhalten; und

Normalisieren der Vor-Korrektur-Reichweite der Anzahl der einzigartigen Sequenzen in jedem Fenster, um den $Z_{CNV}$-Wert der Anzahl der einzigartigen Sequenzen in jedem Fenster zu erhalten und Bestimmen, ob die zu testende schwangere Frau ein chromosomales Fragment mit der Kopiezahlvariation hat, basierend auf der Größenordnung des $Z_{CNV}$-Wertes;

wenn es in den Sequenzierungsdaten Fragmente gibt, die 300kb oder mehr lang sind, und innerhalb der Fragmente, die 300kb oder mehr lang sind, die $Z_{CNV}$-Werte der Anzahlen der einzigartigen Sequenzen in mindestens 80% der gesamten Fenster größer als oder gleich 4 oder weniger als oder gleich -4 sind, dann werden die Fragmente, die 300 kb oder mehr lang sind, als die Fragmente mit Kopiezahlvariationen der zu testenden schwangeren Frau bestimmt;
beinhaltet.

5. Verfahren nach Anspruch 1, wobei für den Schritt des Durchführens eines Z-Tests für jedes Chromosom durch Verwendung der korrigierten Reichweite jedes Chromosoms, um den $Z_{aneu}$-Wert zu erhalten, der $Z_{aneu}$-Wert berechnet wird als:

$$Z_{aneu} = \frac{x' - \overline{x}}{s}$$

wobei $\overline{x}$ die Vor-Korrektur-Reichweite, die von einer bekannten negativen Probenpopulation gemäß einem LOESS-Algorithmus erhalten wird, repräsentiert; s den Standardfehler von $(x' - \overline{x})$ in der negativen Probenpopulation repräsentiert.

6. Vorrichtung zur Detektion von Chromosom-Aneuploidie, die dadurch charakterisiert ist, dass die Vorrichtung die folgenden Module beinhaltet:

ein Sequenzierungsdaten-Detektionsmodul: für das Hochdurchsatz-Sequenzieren der zellfreien DNA aus peripherem Blut einer zu testenden schwangeren Frau, um die Sequenzierungsdaten, die alle der Chromosomen umfasst, zu produzieren;
ein erstes Reichweite-Berechnungsmodul: für das Berechnen einer Reichweitestatistik für alle der Chromosomen anhand der Sequenzierungsdaten durch Segmentieren der Chromosomen in Fenster um eine Vor-Korrektur-Reichweite jedes Chromosoms zu produzieren;
ein $Z_{CNV}$-Wert-Berechnungsmodul: für das Berechnen des $Z_{CNV}$-Werts für die Anzahl an einzigartigen Sequenzen in jedem Fenster der schwangeren Frau;
ein Fragmente-mit-Kopiezahlvariationen-Suchmodul: für das Suchen des Fragments, dass 300 kb oder mehr lang ist und das die $Z_{CNV}$-Werte der Chromosomfragmente von größer als oder gleich 4 oder weniger als oder gleich -4 in mindestens 80% der gesamten Fenster hat, in den Sequenzierungsdaten;
ein Fragmente-mit-Kopiezahlvariationen-Bestimmungsmodul: für das Bestimmen von Fragmenten in den Sequenzierungsdaten, die 300 kb oder mehr lang sind und die $Z_{CNV}$- Werte der Chromosomfragmente von größer als oder gleich 4 oder weniger als oder gleich -4 in mindestens 80% der gesamten Fenster haben, als Fragmente mit Kopiezahlvariationen der schwangeren Frau;
ein erstes $\alpha$-Berechnungsmodul: für das Berechnen eines Parameters a gemäß Formel (1) in dem Fall, dass der Fötus die Fragmente mit Kopiezahlvariationen von der Mutter erbt, wobei der Parameter a die Auswirkung der Fragmente mit Kopiezahlvariationen schwangeren Frau auf die Vor-Korrektur-Reichweite jedes Chromosoms repräsentiert;

$$\alpha = \frac{(m-n) \cdot 2 + n \cdot cn}{m \cdot 2} \quad\ldots\ldots\ldots\ldots\ldots\ldots\ldots\ldots\ldots\ldots (1)$$

wobei in Formel (1) m die effektive Länge des Chromosoms, in dem die Fragmente mit Kopiezahlvariationen vorkommen, repräsentiert, in der Einheit Mb; und n die Länge der Fragmente mit Kopiezahlvariationen der zu testenden schwangeren Frau repräsentiert, in der Einheit Mb; cn die Kopiezahl der Fragmente mit Kopiezahlvariationen, die in der schwangeren Frau gefunden werden, repräsentiert;
ein zweites $\alpha$-Berechnungsmodul: für das Berechnen eines Parameters a gemäß Formel (2) in dem Fall, dass der Fötus die Fragmente mit Kopiezahlvariationen nicht von der Mutter erbt, wobei der Parameter a gemäß Formel (2) berechnet wird:

$$\alpha = \frac{(m-n) \cdot 2 + f \cdot n \cdot 2 + (1-f) \cdot n \cdot cn}{m \cdot 2} \quad\ldots\ldots\ldots\ldots (2)$$

wobei in Formel (2), m die effektive Länge des Chromosoms, in dem die Fragmente mit Kopiezahlvariationen vorkommen, repräsentiert, in der Einheit Mb; und n die Länge der Fragmente mit Kopiezahlvariationen der schwangeren Frau repräsentiert, in der Einheit Mb; cn die Kopiezahl der Fragmente mit Kopiezahlvariationen, die in der schwangeren Frau gefunden werden, repräsentiert; f den Bruchteil der zellfreien fötalen DNA, die in der zellfreien DNA aus dem peripheren Blut der schwangeren Frau existiert, repräsentiert, und der Bruchteil f der zellfreien fötalen DNA als weniger als 50% angenommen wird;

ein Korrektionsmodul: für das Korrigieren der Vor-Korrektur-Reichweite jedes Chromosoms durch Verwendung

$$x^{'} = \frac{\hat{x}}{\alpha}$$

von: um die korrigierte Reichweite jedes Chromosoms zu produzieren; wobei $\hat{x}$ die Vor-Korrektur-Reichweite jedes Chromosoms repräsentiert, und x' die korrigierte Reichweite jedes Chromosoms repräsentiert;

ein zweites Reichweite-Berechnungsmodul: für das Berechnen des $Z_{aneu}$-Wertes jedes Chromosoms durch Verwendung der korrigierten Reichweite jedes Chromosoms;

$Z_{aneu}$-Wert-Bestimmungsmodul: für das Bestimmen, ob der absolute $Z_{aneu}$-Wert größer als oder gleich 3 ist;

ein Chromosom-Aneuploidie-Bestätigungsmodul: für das Bestätigen, dass das Chromosom aneuploid ist, für den Fall, dass der absolute $Z_{aneu}$-Wert größer als oder gleich 3 ist.

7. Vorrichtung nach Anspruch 6, wobei das erste Reichweite-Berechnungsmodul beinhaltet:

ein Chromosom-Fenster-Segmentierungs-Submodul: für das Segmentieren aller der Chromosomen in den Sequenzierungsdaten in Fenster mit gleicher Größe;
ein erstes Reichweite-Berechnungs-Submodul: für das Berechnen einer Reichweitestatistik in der Form von Fenstern mit gleicher Größe, um die Vor-Korrektur-Reichweite jedes Chromosoms zu produzieren.

8. Vorrichtung nach Anspruch 7, wobei die Länge jedes Fensters in dem Chromosom-Fenster-Segmentierungs-Submodul 100 kb ist, und das Überlappungsverhältnis zwischen zwei nebeneinanderliegenden Fenstern 50% ist.

9. Vorrichtung nach Anspruch 6, wobei das $Z_{CNV}$-Wert-Berechnungsmodul beinhaltet:

eine Einzigartige-Sequenz-Zähleinheit: für das Zählen der Anzahl der einzigartigen Sequenzen in jedem Fenster gemäß der Sequenzierungstiefe jeder Sequenz in den Sequenzierungsdaten;
eine Einzigartige-Sequenz-Reichweite-Berechnungseinheit: für das Berechnen der Anzahl der einzigartigen Sequenzen in jedem Fenster gemäß dem GC-Gehalt und der Zuordnungsrate jedes Chromosoms, um die Vor-Korrektur-Reichweite der Anzahl der einzigartigen Sequenzen in jedem Fenster zu erhalten; und
eine Einzigartige-Sequenz-$Z_{CNV}$-Wert-Berechnungseinheit: für das Normalisieren der Vor-Korrektur-Reichweite der Anzahl der einzigartigen Sequenzen in jedem Fenster, um den $Z_{CNV}$-Wert der Anzahl der einzigartigen Sequenzen in jedem Fenster zu erhalten.

10. Vorrichtung nach Anspruch 6, wobei in dem zweiten Reichweite-Berechnungsmodul der $Z_{aneu}$-Wert berechnet wird als:

$$Z_{aneu} = \frac{x^{'} - \overline{x}}{s}$$

wobei $\overline{x}$ die Vor-Korrektur-Reichweite, die von einer bekannten negativen Probenpopulation gemäß einem LOESS-Algorithmus erhalten wird, ist; s den Standardfehler von ($x'$-$\overline{x}$) in der negativen Probenpopulation repräsentiert.

11. Kit zur Detektion von Chromosom-Aneuploidie, welches dadurch charakterisiert ist, dass das Kit beinhaltet:

die Detektionsreagenzien und ein Detektionsgerät: für das Hochdurchsatz-Sequenzieren der zellfreien DNA aus peripherem Blut einer zu testenden schwangeren Frau, um Sequenzierungsdaten, die alle der Chromosomen beinhalten, zu produzieren;
ein erstes Reichweite-Berechnungsgerät: für das Berechnen einer Reichweitestatistik für alle der Chromosomen anhand der Sequenzierungsdaten durch Segmentieren der Chromosomen in Fenster um eine Vor-Korrektur-Reichweite für jedes Chromosom zu produzieren;
ein $Z_{CNV}$-Wert-Berechnungsgerät: für das Durchführen eines Z-Tests an der Anzahl an einzigartigen Sequenzen in jedem Fenster der zu testenden schwangeren Frau, um den $Z_{CNV}$-Wert zu erhalten;

ein Fragmente-mit-Kopiezahlvariationen-Suchmodul: für das Suchen der Fragmente in den Sequenzierungsdaten, die 300 kb oder mehr lang sind und die $Z_{CNV}$-Werte der chromosomalen Fragmente von größer als oder gleich 4 oder weniger als oder gleich -4 in mindestens 80% der gesamten Fenster haben;

ein Fragmente-mit-Kopiezahlvariationen-Bestimmungsmodul: für das Erhalten der Fragmente mit Kopiezahlvariationen der zu testenden schwangeren Frau basierend auf der Größenordnung des $Z_{CNV}$-Wertes;

ein erstes α-Berechnungsgerät: für das Berechnen eines Parameters a gemäß Formel (1) in dem Fall, dass der Fötus die Fragmente mit Kopiezahlvariationen von der Mutter erbt, wobei der Parameter a die Auswirkung der Fragmente mit Kopiezahlvariationen jedes Chromosoms der zu testenden schwangeren Frau auf die Vor-Korrektur-Reichweite repräsentiert;

$$\alpha = \frac{(m-n)\cdot 2 + n\cdot cn}{m\cdot 2} \quad\dots\dots\dots\dots\dots\dots\dots\dots\dots\dots\dots\;(1)$$

wobei m die effektive Länge des Chromosoms, in dem die Fragmente mit Kopiezahlvariationen vorkommen, repräsentiert, in der Einheit Mb; und n die Länge der Fragmente mit Kopiezahlvariationen der zu testenden schwangeren Frau repräsentiert, in der Einheit Mb; cn die Kopiezahl der Fragmente mit Kopiezahlvariationen, die in der zu testenden schwangeren Frau gefunden werden, repräsentiert;

ein zweites α-Berechnungsgerät: für das Berechnen eines Parameters a gemäß Formel (2) in dem Fall, dass der Fötus die Fragmente mit Kopiezahlvariationen nicht von der Mutter erbt, wobei der Parameter a gemäß Formel (2) berechnet wird:

$$\alpha = \frac{(m-n)\cdot 2 + f\cdot n\cdot 2 + (1-f)\cdot n\cdot cn}{m\cdot 2} \quad\dots\dots\dots\dots\;(2)$$

wobei m die effektive Länge des Chromosoms, in dem die Fragmente mit Kopiezahlvariationen vorkommen, repräsentiert, in der Einheit Mb; und n die Länge der Fragmente mit Kopiezahlvariationen der schwangeren Frau repräsentiert, in der Einheit Mb; cn die Kopiezahl der Fragmente mit Kopiezahlvariationen, die in der schwangeren Frau gefunden werden, repräsentiert; f den Bruchteil der zellfreien fötalen DNA, die in der zellfreien DNA aus dem peripheren Blut der schwangeren Frau existiert, repräsentiert, und der Bruchteil f der zellfreien fötalen DNA als weniger als 50% angenommen wird;

ein Korrektionsgerät: für das Korrigieren der Vor-Korrektur-Reichweite jedes Chromosoms durch Verwendung

$$x' = \frac{\hat{x}}{\alpha}$$

von: um die korrigierte Reichweite jedes Chromosoms zu produzieren; wobei $\hat{x}$ die Vor-Korrektur-Reichweite jedes Chromosoms repräsentiert, und x' die korrigierte Reichweite jedes Chromosoms repräsentiert;

ein zweites Reichweite-Berechnungsgerät: für das Berechnen des $Z_{aneu}$-Wertes jedes Chromosoms durch Verwendung der korrigierten Reichweite jedes Chromosoms;

$Z_{aneu}$-Wert-Bestimmungsgerät: für das Bestimmen, ob der absolute $Z_{aneu}$-Wert größer als oder gleich 3 ist;

ein Chromosom-Aneuploidie-Bestätigungsgerät: für das Bestätigen, dass das Chromosom aneuploid ist, für den Fall, dass der absolute $Z_{aneu}$-Wert größer als oder gleich 3 ist.

12. Kit nach Anspruch 11, wobei das erste Reichweite-Berechnungsgerät beinhaltet:

eine Chromosom-Fenster-Segmentierungskomponente: für das Segmentieren aller der Chromosomen in den Sequenzierungsdaten in Fenster mit gleicher Größe;

eine erste Reichweite-Berechnungskomponente: für das Berechnen einer Reichweitestatistik in der Form von Fenstern mit gleicher Größe, um die Vor-Korrektur-Reichweite jedes Chromosoms zu produzieren.

13. Kit nach Anspruch 12, wobei die Länge jedes Fensters in der Chromosom-Fenster-Segmentierungskomponente 100 kb ist, und das Überlappungsverhältnis zwischen zwei nebeneinanderliegenden Fenstern 50% ist.

14. Kit nach Anspruch 11, wobei das $Z_{CNV}$-Wert-Berechnungsgerät beinhaltet:

eine Einzigartige-Sequenz-Zählkomponente: für das Zählen der Anzahl an einzigartigen Sequenzen in jedem Fenster gemäß der Sequenzierungstiefe jeder Sequenz in den Sequenzierungsdaten;

eine Einzigartige-Sequenz-Reichweite-Berechnungskomponente: für das Berechnen der Anzahl der einzigartigen Sequenzen in jedem Fenster gemäß dem GC-Gehalt und der Zuordnungsrate jedes Chromosoms, um die Vor-Korrektur-Reichweite der Anzahl der einzigartigen Sequenzen in jedem Fenster zu erhalten; und

eine Einzigartige-Sequenz-$Z_{CNV}$-Wert-Berechnungskomponente: für das Normalisieren der Vor-Korrektur-Reichweite der Anzahl der einzigartigen Sequenzen in jedem Fenster um den $Z_{CNV}$-Wert der Anzahl der einzigartigen Sequenzen in jedem Fenster zu erhalten.

**15.** Kit nach Anspruch 11, wobei in dem zweiten Reichweite-Berechnungsgerät der $Z_{aneu}$-Wert berechnet wird als:

$$Z_{aneu} = \frac{x^{'} - \overline{x}}{s}$$

wobei $\overline{x}$ die Vor-Korrektur-Reichweite, die von einer bekannten negativen Probenpopulation gemäß einem LOESS-Algorithmus erhalten wird, ist; s den Standardfehler von ($x'$-$\overline{x}$) in der negativen Probenpopulation repräsentiert.

**Revendications**

1. Procédé de détection d'une aneuploïdie chromosomique, lequel est **caractérisé en ce que** le procédé inclut les étapes suivantes de :

   séquençage à haut débit de l'ADN exempt de cellules sanguines périphériques provenant d'une femme enceinte à tester pour produire des données de séquençage comprenant la totalité des chromosomes ;

   calcul de statistiques de couverture pour la totalité des chromosomes avec les données de séquençage en segmentant les chromosomes en fenêtres de manière à produire une couverture de précorrection de chaque chromosome ;

   exécution d'un test Z sur le nombre de séquences uniques dans chaque fenêtre de la femme enceinte pour produire une valeur $Z_{CNV}$ et ensuite localisation de fragments chromosomiques à variations du nombre de copies de la femme enceinte sur la base de la magnitude de la valeur $Z_{CNV}$ ; sachant que les fragments chromosomiques à variations du nombre de copies de la femme enceinte sont de 300 kb ou plus en longueur et ont des valeurs $Z_{CNV}$ des fragments chromosomiques supérieures ou égales à 4 ou inférieures ou égales à -4 dans au moins 80 % des fenêtres totales à l'intérieur des fragments qui sont de 300 kb ou plus en longueur, correction de la couverture de précorrection de chaque chromosome moyennant l'impact des fragments à variations du nombre de copies de la femme enceinte sur la couverture de précorrection de chaque chromosome pour produire la couverture corrigée de chaque chromosome ; et

   exécution d'un test Z de chaque chromosome moyennant la couverture corrigée de chaque chromosome pour obtenir une valeur $Z_{aneu}$, et détermination d'une aneuploïdie chromosomique en fonction de si la valeur absolue de $Z_{aneu}$ est supérieure ou égale à 3 ; sachant que si la valeur absolue de $Z_{aneu}$ est supérieure ou égale à 3, l'aneuploïdie chromosomique est déterminée ;

   sachant que l'impact des fragments à variations du nombre de copies de la femme enceinte à tester sur la couverture de précorrection de chaque chromosome est représenté par un paramètre $\alpha$,

   lorsque le foetus hérite des fragments à variations du nombre de copies de la mère, le paramètre a est calculé selon la formule (1) :

$$\alpha = \frac{(m-n)\cdot 2 + n \cdot cn}{m \cdot 2} \tag{1}$$

   lorsque le foetus n'hérite pas des fragments à variations du nombre de copies de la mère, le paramètre $\alpha$ est calculé selon la formule (2) :

$$\alpha = \frac{(m-n)\cdot 2 + f \cdot n \cdot 2 + (1-f)\cdot n \cdot cn}{m \cdot 2} \tag{2}$$

sachant que dans la formule (1) et la formule (2), m représente la longueur effective du chromosome dans lequel les fragments à variations du nombre de copies survient,
dans l'unité de Mb ; et n représente la longueur des fragments à variations du nombre de copies de la femme enceinte à tester, dans l'unité de Mb ; cn représente le nombre de copies des fragments à variations du nombre de copies trouvés chez la femme enceinte à tester ;
sachant que dans la formule (2), f représente la fraction de l'ADN foetal exempt de cellules existant dans l'ADN exempt de cellules sanguines périphériques de la femme enceinte à tester, et la fraction f de l'ADN foetal exempt de cellules est estimé être inférieur à 50 % ;
correction de la couverture de précorrection de chaque chromosome moyennant

$$x' = \frac{\hat{x}}{\propto},$$

sachant que $\hat{x}$ représente la couverture de précorrection de chaque chromosome et $x'$ représente la couverture corrigée de chaque chromosome.

2. Le procédé de la revendication 1, sachant que les statistiques de couverture sont calculées en segmentant la totalité des chromosomes dans les données de séquençage en fenêtres de tailles égales de manière à produire la couverture de précorrection de chaque chromosome.

3. Le procédé selon la revendication 2, sachant que la longueur de chaque fenêtre est de 100 kb et le rapport de chevauchement entre deux fenêtres adjacentes est de 50 %.

4. Le procédé selon la revendication 1, sachant que l'étape d'exécution d'un test Z sur le nombre de séquences uniques dans chaque fenêtre de la femme enceinte à tester pour produire la valeur $Z_{CNV}$ puis localisation de fragments chromosomiques à variations du nombre de copies de la femme enceinte à tester sur la base de la magnitude de la valeur $Z_{CNV}$ inclut en outre les étapes de :

   comptage du nombre des séquences uniques dans chaque fenêtre selon la profondeur de séquençage de chaque séquence dans les données de séquençage ;
   calcul du nombre des séquences uniques dans chaque fenêtre selon la teneur en GC et le taux de mappage de chaque chromosome pour obtenir la couverture de précorrection du nombre des séquences uniques dans chaque fenêtre ; et
   normalisation de la couverture de précorrection du nombre des séquences uniques dans chaque fenêtre pour obtenir la valeur $Z_{CNV}$ du nombre des séquences uniques dans chaque fenêtre et fait de déterminer si la femme enceinte à tester présente un fragment chromosomique avec la variation du nombre de copies sur la base de la magnitude de la valeur $Z_{CNV}$ ;
   s'il existe des fragments qui sont de 300 kb ou plus en longueur dans les données de séquençage, et à l'intérieur des fragments qui sont de 300 kb ou plus en longueur, les valeurs $Z_{CNV}$ des nombres des séquences uniques dans au moins 80 % des fenêtres totales sont supérieures ou égales à 4 ou inférieures ou égales à -4, alors les fragments qui sont de 300 kb ou plus en longueur sont déterminés pour être les fragments à variations du nombre de copies de la femme enceinte à tester.

5. Le procédé selon la revendication 1, sachant que pour l'étape d'exécution d'un test Z pour chaque chromosome moyennant la couverture corrigée de chaque chromosome pour obtenir la valeur $Z_{aneu}$, la valeur $Z_{aneu}$ est calculée comme étant :

$$Z_{aneu} = \frac{x' - \overline{x}}{s}$$

sachant que $\overline{x}$ représente la couverture de précorrection obtenue à partir d'une population échantillon négative connue selon un algorithme de LOESS ; s représente l'erreur standard de ($x'$ - $\overline{x}$) dans la population échantillon négative.

6. Appareil de détection d'une aneuploïdie chromosomique, lequel est **caractérisé en ce que** l'appareil inclut les modules suivants :

un module de détection de données de séquençage : pour effectuer un séquençage à haut débit de l'ADN exempt de cellules sanguines périphériques provenant d'une femme enceinte à tester pour produire les données de séquençage comprenant la totalité des chromosomes ;

un premier module de calcul de couverture : pour calculer des statistiques de couverture de la totalité des chromosomes avec les données de séquençage en segmentant les chromosomes en fenêtres de manière à produire une couverture de précorrection de chaque chromosome ;

un module de calcul de valeur $Z_{CNV}$ : pour calculer la valeur $Z_{CNV}$ sur le nombre de séquences uniques dans chaque fenêtre de la femme enceinte ;

un module de recherche de fragments à variations du nombre de copies : pour rechercher le fragment qui est de 300 kb ou plus en longueur dans les données de séquençage et dont les valeurs $Z_{CNV}$ des fragments chromosomiques sont supérieures ou égales à 4 ou inférieures ou égales à -4 dans au moins 80 % des fenêtres totales ;

un module de détermination de fragments à variations du nombre de copies : pour déterminer des fragments dans les données de séquençage qui sont de 300 kb ou plus en longueur et dont les valeurs $Z_{CNV}$ des fragments chromosomiques sont supérieures ou égales à 4 ou inférieures ou égales à -4 dans au moins 80 % des fenêtres totales en tant que les fragments à variations du nombre de copies de la femme enceinte ;

un premier module de calcul de $\alpha$ : pour calculer un paramètre $\alpha$ selon la formule (1) dans le cas où le foetus hérite des fragments à variations du nombre de copies de la mère, sachant que le paramètre $\alpha$ représente l'impact des fragments à variations du nombre de copies de la femme enceinte sur la couverture de précorrection de chaque chromosome ;

$$\alpha = \frac{(m-n)\cdot 2 + n \cdot cn}{m \cdot 2} \tag{1}$$

sachant que dans la formule (1), m représente la longueur effective du chromosome dans lequel les fragments à variations du nombre de copies survient, dans l'unité de Mb ; et n représente la longueur des fragments à variations du nombre de copies de la femme enceinte à tester, dans l'unité de Mb ; cn représente le nombre de copies des fragments à variations du nombre de copies trouvés chez la femme enceinte ;

un deuxième module de calcul de $\alpha$ : pour calculer un paramètre $\alpha$ selon la formule (2) dans le cas où le foetus n'hérite pas des fragments à variations du nombre de copies de la mère, sachant que le paramètre $\alpha$ est calculé selon la formule (2)

$$\alpha = \frac{(m-n)\cdot 2 + f \cdot n \cdot 2 + (1-f)\cdot n \cdot cn}{m \cdot 2} \tag{2}$$

sachant que dans la formule (2), m représente la longueur effective du chromosome dans lequel les fragments à variations du nombre de copies survient, dans l'unité de Mb ; et n représente la longueur des fragments à variations du nombre de copies de la femme enceinte, dans l'unité de Mb ; cn représente le nombre de copies des fragments à variations du nombre de copies trouvés chez la femme enceinte ; f représente la fraction de l'ADN foetal exempt de cellules existant dans l'ADN exempt de cellules sanguines périphériques de la femme enceinte, et la fraction f de l'ADN foetal exempt de cellules est estimé être inférieur à 50 % ;

un module de correction : pour corriger la couverture de précorrection de chaque chromosome moyennant :

$$x' = \frac{\hat{x}}{\alpha}$$

pour produire la couverture corrigée de chaque chromosome ; sachant que $\hat{x}$ représente la couverture de précorrection de chaque chromosome et x' représente la couverture corrigée de chaque chromosome ;

un deuxième module de calcul de couverture : pour calculer la valeur $Z_{aneu}$ de chaque chromosome moyennant la couverture corrigée de chaque chromosome ;

un module de détermination de valeur $Z_{aneu}$ : pour déterminer si la valeur absolue $Z_{aneu}$ est supérieure ou égale à 3 ;

un module de confirmation d'aneuploïdie chromosomique : pour confirmer que le chromosome est aneuploïde dans le cas où la valeur absolue $Z_{aneu}$ est supérieure ou égale à 3.

**7.** L'appareil selon la revendication 6, sachant que le premier module de calcul de couverture inclut :

un sous-module de segmentation en fenêtres de chromosomes : pour segmenter la totalité des chromosomes dans les données de séquençage en fenêtres de taille égale ;

un premier sous-module de calcul de couverture : pour calculer des statistiques de couverture sous la forme de fenêtres de taille égale pour produire la couverture de précorrection de chaque chromosome.

**8.** L'appareil selon la revendication 7, sachant que la longueur de chaque fenêtre dans le sous-module de segmentation en fenêtres de chromosomes est de 100 kb, et le rapport de chevauchement entre deux fenêtres adjacentes est de 50 %.

**9.** L'appareil selon la revendication 6, sachant que le module de calcul de valeur $Z_{CNV}$ inclut :

une unité de comptage de séquences uniques : pour compter le nombre des séquences uniques dans chaque fenêtre selon la profondeur de séquençage de chaque séquence dans les données de séquençage ;

une unité de calcul de couverture de séquences uniques : pour calculer le nombre des séquences uniques dans chaque fenêtre selon la teneur en GC et le taux de mappage de chaque chromosome pour obtenir la couverture de précorrection du nombre des séquences uniques dans chaque fenêtre ; et

une unité de calcul de valeur $Z_{CNV}$ de séquences uniques : pour normaliser la couverture de précorrection du nombre des séquences uniques dans chaque fenêtre pour obtenir la valeur $Z_{CNV}$ du nombre des séquences uniques dans chaque fenêtre.

**10.** L'appareil selon la revendication 6, sachant que dans le deuxième module de calcul de couverture, la valeur $Z_{aneu}$ est calculée comme étant :

$$Z_{aneu} = \frac{x' - \overline{x}}{s}$$

sachant que *x* est la couverture de précorrection obtenue à partir d'une population échantillon négative connue selon un algorithme de LOESS ; s représente l'erreur standard de $(x' - \overline{x})$ dans la population échantillon négative.

**11.** Kit de détection d'une aneuploïdie chromosomique, lequel est **caractérisé en ce que** le kit inclut :

les réactifs de détection et un dispositif de détection : pour effectuer un séquençage à haut débit de l'ADN exempt de cellules sanguines périphériques provenant d'une femme enceinte à tester pour produire les données de séquençage comprenant la totalité des chromosomes ;

un premier dispositif de calcul de couverture : pour calculer des statistiques de couverture de la totalité des chromosomes avec les données de séquençage en segmentant les chromosomes en fenêtres de manière à produire une couverture de précorrection de chaque chromosome ;

un dispositif de calcul de valeur $Z_{CNV}$ : pour effectuer un test Z sur le nombre de séquences uniques dans chaque fenêtre de la femme enceinte à tester pour obtenir la valeur $Z_{CNV}$ ;

un dispositif de recherche de fragments à variations du nombre de copies : pour rechercher les fragments dans les données de séquençage qui sont de 300 kb ou plus en longueur et dont les valeurs $Z_{CNV}$ des fragments chromosomiques sont supérieures ou égales à 4 ou inférieures ou égales à -4 dans au moins 80 % des fenêtres totales ;

un dispositif de détermination de fragments à variations du nombre de copies : pour obtenir les fragments à variations du nombre de copies de la femme enceinte à tester sur la base de la magnitude de la valeur $Z_{CNV}$ ;

un premier dispositif de calcul de $\alpha$ : pour calculer un paramètre $\alpha$ selon la formule (1) dans le cas où le foetus hérite des fragments à variations du nombre de copies de la mère, sachant que le paramètre $\alpha$ représente l'impact des fragments à variations du nombre de copies de la femme enceinte à tester sur la couverture de précorrection de chaque chromosome ;

$$\alpha = \frac{(m-n)\cdot 2 + n \cdot cn}{m \cdot 2} \qquad (1)$$

sachant que m représente la longueur effective du chromosome dans lequel les fragments à variations du nombre de copies surviennent, dans l'unité de Mb ; et n représente la longueur des fragments à variations du nombre de copies de la femme enceinte à tester, dans l'unité de Mb ; cn représente le nombre de copies des fragments à variations du nombre de copies trouvés chez la femme enceinte ;
un deuxième dispositif de calcul de $\alpha$ : pour calculer un paramètre $\alpha$ selon la formule (2) dans le cas où le foetus n'hérite pas des fragments à variations du nombre de copies de la mère, sachant que le paramètre $\alpha$ est calculé selon la formule (2)

$$\alpha = \frac{(m-n)\cdot 2 + f \cdot n \cdot 2 + (1-f)\cdot n \cdot cn}{m \cdot 2} \qquad (2)$$

sachant que m représente la longueur effective du chromosome dans lequel les fragments à variations du nombre de copies survient, dans l'unité de Mb ; et n représente la longueur des fragments à variations du nombre de copies de la femme enceinte, dans l'unité de Mb ; cn représente le nombre de copies des fragments à variations du nombre de copies trouvés chez la femme enceinte ; f représente la fraction de l'ADN foetal exempt de cellules existant dans l'ADN exempt de cellules sanguines périphériques de la femme enceinte, et la fraction f de l'ADN foetal exempt de cellules est estimé être inférieur à 50 % ;
un dispositif de correction : pour corriger la couverture de précorrection de chaque chromosome moyennant :

$$x' = \frac{\hat{x}}{\alpha}$$

pour produire la couverture corrigée de chaque chromosome ; sachant que $\hat{x}$ représente la couverture de précorrection de chaque chromosome et x' représente la couverture corrigée de chaque chromosome ;
un deuxième dispositif de calcul de couverture : pour calculer la valeur $Z_{aneu}$ de chaque chromosome moyennant la couverture corrigée de chaque chromosome ;
un dispositif de détermination de valeur $Z_{aneu}$ : pour déterminer si la valeur absolue $Z_{aneu}$ est supérieure ou égale à 3 ;
un dispositif de confirmation d'aneuploïdie chromosomique : pour confirmer une aneuploïdie chromosomique dans le cas où la valeur absolue $Z_{aneu}$ est supérieure ou égale à 3.

12. Le kit selon la revendication 11, sachant que le premier dispositif de calcul de couverture inclut :

un composant de segmentation en fenêtres de chromosomes : pour segmenter la totalité des chromosomes dans les données de séquençage en fenêtres de taille égale ;
un premier composant de calcul de couverture : pour calculer les statistiques de couverture sous la forme de fenêtres de taille égale pour produire la couverture de précorrection de chaque chromosome.

13. Le kit selon la revendication 12, sachant que la longueur de chaque fenêtre dans le composant de segmentation en fenêtres de chromosomes est de 100 kb, et le rapport de chevauchement entre deux fenêtres adjacentes est de 50 %.

14. Le kit selon la revendication 11, sachant que le dispositif de calcul de valeur $Z_{CNV}$ inclut :

un composant de comptage de séquences uniques : pour compter le nombre des séquences uniques dans chaque fenêtre selon la profondeur de séquençage de chaque séquence dans les données de séquençage ;
un composant de calcul de couverture de séquences uniques : pour calculer le nombre des séquences uniques dans chaque fenêtre selon la teneur en GC et le taux de mappage de chaque chromosome pour obtenir la couverture de précorrection du nombre des séquences uniques dans chaque fenêtre ; et
un composant de calcul de valeur $Z_{CNV}$ de séquences uniques : pour normaliser la couverture de précorrection du nombre des séquences uniques dans chaque fenêtre pour obtenir la valeur $Z_{CNV}$ du nombre des séquences uniques dans chaque fenêtre.

15. Le kit selon la revendication 11, sachant que dans le deuxième dispositif de calcul de couverture, la valeur $Z_{aneu}$ est calculée comme étant :

$$Z_{aneu} = \frac{x' - \overline{x}}{s}$$

sachant que $x$ est la couverture de précorrection obtenue à partir d'une population échantillon négative connue selon un algorithme de LOESS ; s représente l'erreur standard de $(x' \overline{x})$ dans la population échantillon négative.

High-throughput sequencing of the peripheral blood cell-free DNA from a pregnant woman to be tested to produce sequencing data comprising all of the chromosomes

Calculating coverage statistics for all of the chromosomes with the sequencing data by segmenting the chromosomes into windows so as to produce a pre-correction coverage for the each chromosome

Calculating a $Z_{CNV}$ value on the number of unique sequence in the each window of the pregnant woman and then locating chromosomalfragment with the copy number variation of the pregnant woman on the basis of the magnitude of the $Z_{CNV}$ value

Correcting the pre-correction coverage of the each chromosome by utilizing the impact of the fragment with copy number variation of the pregnant woman on the pre-correction coverage of the each chromosome to produce the corrected coverage for the each chromosome

Calculating $Z_{aneu}$ value for the each chromosome by using the corrected coverage of the each chromosome, and determining whether the chromosome has an aneuploidy based on whether the absolute value of $Z_{aneu}$ is greater than or equal to 3; wherein when the absolute value of $Z_{aneu}$ is greater than or equal to 3, then it is determined that the chromosome has an aneuploidy

fig.1

fig.2

Chromosome 13

Size of the repeat area of the pregnant woman(Mb)

## fig.3A

Chromosome 18

Size of the repeat area of the pregnant woman(Mb)

## fig.3B

Chromosome 21

Size of the repeat area of the pregnant woman(Mb)

fig.3C

Before correcting          After correcting

fig.4

fig.5

fig.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BAYINDIR et al.** *Eur J Hum Gen,* 2015, vol. 23 (10), 1286-129

- *PLOS ONE,* 2014, vol. 9, 71-7